# EUROPEAN PATENT APPLICATION

(11) **EP 4 678 192 A1**
(43) Date of publication of application: **14.01.2026**
(21) Application number: 24769906.9
(22) Date of filing: 11.03.2024
(51) Int. Cl.: A61K 47/68, A61K 47/65, C08B 37/00, A61K 38/07, C07K 16/28, C07K 16/24, C07K 16/22, A61K 39/44, A61K 31/542, A61P 35/00

(54) **DOUBLE SITE-DIRECTED ANTIBODY-FUNCTIONAL MOLECULAR CONJUGATE, AND PREPARATION METHOD THEREFOR AND USE THEREOF**

(30) Priority: 10.03.2023 CN 202310229547
(71) Applicant: Shanghai Institute of Materia Medica, Chinese Academy of Sciences, Shanghai 201203 (CN)
(72) Inventor: HUANG, Wei, Shanghai 201203 (CN); TANG, Feng, Shanghai 201203 (CN); SHI, Wei, Shanghai 201203 (CN); TANG, Caihong, Shanghai 201203 (CN); XIA, Fei, Shanghai 201203 (CN); LIU, Zhaojun, Shanghai 201203 (CN)
(74) Representative: Witte, Weller & Partner Patentanwälte mbB
(86) International application number: PCT/CN2024/080954
(87) International publication number: WO 2024/188213

(57) **Abstract**

A double site-directed antibody-functional molecular conjugate, and a preparation method therefor and the use thereof. The double site-directed antibody-functional molecular conjugate comprises an antibody, linking fragments 1 and 2 and a functional molecule, wherein the functional molecule is coupled to a conserved glycosylation site in the Fc region of the antibody by the linking fragment 1 and is coupled to a lysine site at position 246 or 248 of the antibody by the linking fragment 2. The double site-directed antibody-functional molecular conjugate has good stability, cytotoxicity and in-vivo activity, has good druggability, etc., and can be used in the preparation of a drug, diagnostic and therapeutic reagents and a kit for treating tumors, diseases caused by inflammation, diseases caused by a virus infection and immune diseases. The preparation method has the advantages of simple operation, few purification steps, etc., and has a good druggability and is easy to industrialize.

## Description

### Technical Field

The present invention relates to the fields of medicinal chemistry and biotechnological drugs, and in particular to a dual-site directed antibody-functional molecule conjugate, and a preparation method and use thereof.

### Background Art

Antibody-Drug Conjugates (ADCs), which consist of an antibody, a cytotoxins, and a linker, deliver a cytotoxin to tumor tissue through the antibody, achieving targeted delivery of cytotoxin and thus exerting anti-tumor activity. As demonstrated by extensive data, site-directed ADCs have a superior therapeutic index compared to randomly conjugated ADCs. The Drug-to-Antibody Ratio (DAR) represents the average number of toxins conjugated on the antibody. The DAR value is crucial to the therapeutic efficacy of ADCs. A lower DAR value may affect the therapeutic effect of the ADC, while a higher DAR value increases the hydrophobicity of the ADC, affecting its pharmacokinetic behavior in vivo. Therefore, the structural design of ADCs should comprehensively consider the antibody, toxin, linker, and conjugation site to determine an appropriate DAR value. For example, for some cytotoxins that are less toxic, ADCs with a high DAR value may be prepared by incorporating a hydrophilic moiety into the drug-linker, thereby achieving an improved killing effect.

In addition, the concept of dual-drug ADCs has emerged in recent years, aiming to achieve a 1+1>2 effect. However, both ADC compounds having specific DAR value and dual-drug ADCs require specific preparation methods. Stephen Caddick et al. reported preparation of dual-drug ADC compounds by introducing linear alkyne and ring-strained alkyne structures into an antibody through a cysteine bridging technique, and further introducing two different toxin molecules through bioorthogonal reaction. Christoph Rader et al. prepare dual-drug ADC compounds by combining THIOMAB and SELENOMAB techniques. Matthew R. Levengood et al. prepared dual-drug ADC compounds with good anti-tumor activities by introducing two thiol groups containing different protecting groups into an antibody, and then deprotecting separately and conjugating to different toxin molecules. Nazzareno Dimasi et al. synthesized a trifunctional linker structure containing a maleimide structure, an alkyne group, and a ketose group, and finally prepared dual-drug ADC compounds.

The above works provide multiple strategies for the preparation of high-DAR ADCs and dual-drug ADCs. These strategies typically require the design of multi-branched structures, the use of various genetic engineering techniques, the complex enzyme combinations, or the protein-protein conjugation. Most of the strategies may introduce multiple hydrophobic structures or result in low conjugation efficiency, which results in, on the one hand, complex preparation method for dual-drug antibody-functional molecule conjugates that is difficult to be industrialized; and on the other hand, high local hydrophobicity and poor stability of the prepared drugs.

Therefore, there is still a need to provide a dual-site-directed antibody-functional molecule conjugate with a new structure, as well as a simple and efficient preparation method thereof.

### Summary of the Invention

Therefore, the inventors of the present disclosure have developed a dual-site antibody-functional molecule conjugate. Antibody-functional molecule conjugates with specific DAR values were prepared by combining a saccharide chain-directed technique with a targeting peptide-mediated site-directed modification to achieve site-specific and quantitative introduction of functional molecules at the glycosylation sites and the lysine 246 or 248 of the antibody. The preparation method of the dual-site directed ADC compound provided by the present disclosure is simple and efficient, and the prepared ADCs have novel molecular structures with good stability and activity in vitro and in vivo.

It is an object of the present invention to provide a dual-site directed antibody-functional molecule conjugate.

It is another object of the present invention to provide a method for preparing the dual-site directed antibody-functional molecule conjugate.

It is another object of the present invention to provide the use of the dual-site directed antibody-functional molecule conjugate in anti-tumor and the like.

According to one embodiment of the present invention, provided is a dual-site directed antibody-functional molecule conjugate comprising an antibody, linker fragments 1 and 2, and a functional molecule; wherein the functional molecule is conjugated to a conserved glycosylation site in the Fc region of the antibody via the linker fragment 1, and to the lysine 246 or 248 of the antibody via the linker fragment 2.

According to one embodiment of the present invention, the functional molecule is conjugated to the conserved glycosylation site in the Fc region of the antibody via the linker fragment 1, and to the lysine site 248 of the antibody via the linker fragment 2.

According to one embodiment of the present invention,
the antibody is selected from the group consisting of humanized antibodies comprising an Fc region and other animal-origin antibodies comprising an Fc region;
the functional molecules are the same as or different from each other and are independently selected from the group consisting of reactive functional groups, fluorescent groups, drugs, toxins, radioactive structures, and lysosomal targeting peptides;
the linker fragment 1 comprises a linker and a saccharide structure, wherein the saccharide structure is linked to the conserved glycosylation site in the Fc region of the antibody, and the linker is linked to the functional molecule;
the linker fragment 2 comprises a linker and fragment A, wherein fragment A is linked to the lysine 246 or 248 of the antibody, and the linker is linked to the functional molecule;
wherein, the saccharide structure represents a structure of a monosaccharide, a disaccharide, an oligosaccharide or a branched saccharide; the fragment A represents an amide structure or a triazole structure; at the glycosylation site of the antibody Fc domain, the linker represents any structure that can link the saccharide structure and the functional molecule; and at the lysine 246 or 248 of the antibody, the linker represents any structure that can link the fragment A and the functional molecule.

In the present invention, more specifically:
the antibody is selected from the group consisting of antibodies of different IgG subtypes, monoclonal antibodies, bifunctional or multifunctional antibodies, polyclonal antibodies, and functional antibodies of different species; for example, trastuzumab (Herceptin), rituximab, pertuzumab, panitumumab, toripalimab, nivolumab, mouse-origin antibodies, rabbit-origin antibodies, and goat-origin antibodies;
in the functional molecule, the reactive functional group is selected from the group consisting of an azide group, a tetrazine group, a TCO group, an alkynyl structure, an aldehyde structure, a carbonyl structure, a hydroxylamine structure, an isothiocyanate, an amino group, a carboxyl group, a mercapto group, an alkenyl group, a maleimide, an acylhydrazone structure, and the like;
the fluorescent group is selected from the group consisting of biotin, FITC, rhodamine, Cy3, Cy5, etc.;
the drug or toxin is selected from the group consisting of MMAE, MMAF, Dxd, Dx8951, SN38, DM1, DM4, PBD, PBD dimer, eribulin, duocarmycin and derivatives of these drugs;
the radioactive structure is selected from the group consisting of ⁶⁸Ga, ¹⁸F, ⁸⁹Zr, etc.;
the lysosomal targeting peptide is SignalTAC.

According to one embodiment of the present invention, the dual-site directed antibody-functional molecule conjugate is represented by the following formula I: 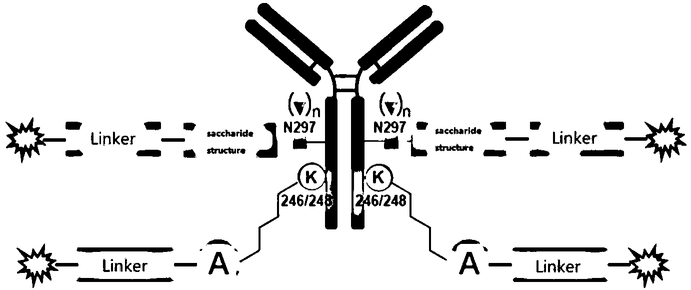 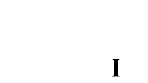
in Formula I,
the "Y"-shaped structure in the center is an antibody;
▼ represents a core fucose structure;
n is 0 or 1;
the linker represents a PEG chain, a carbon chain, a cleavable linker, etc.;
the saccharide structure represents a monosaccharide, a disaccharide, an oligosaccharide or a branched saccharide structure;
the fragment A represents an amide structure or a triazole structure;
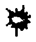 represents a functional molecule, and the functional molecules at the glycosylation site and the lysine site can be the same or different, and one or more functional molecules can be conjugated.

According to one aspect of the present invention, the dual-site directed antibody-functional molecule conjugate of Formula I is represented by the following Formula II: 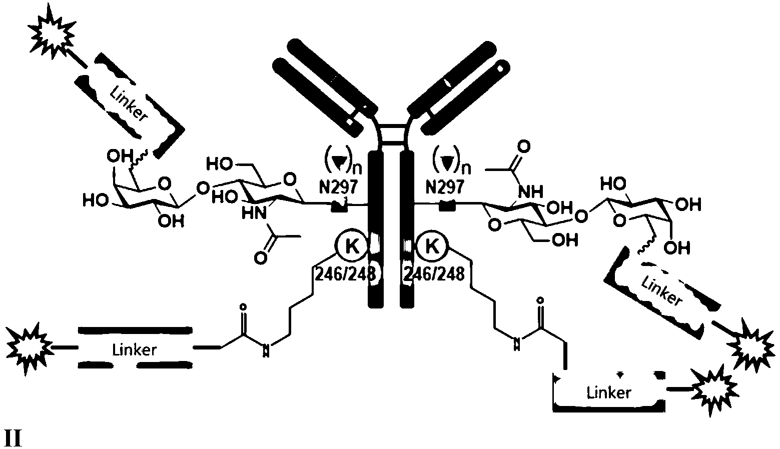 in Formula II, the definitions of the various moieties are the same as above.

According to one aspect of the present invention, the dual-site-directed antibody-functional molecule conjugate of Formula II is selected from the group consisting of: 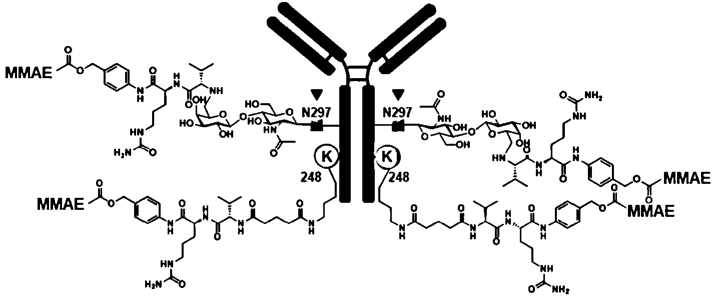 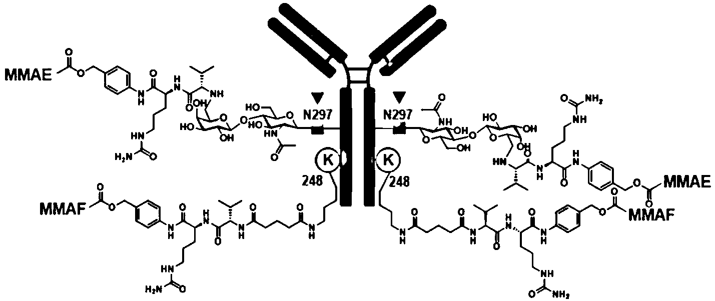 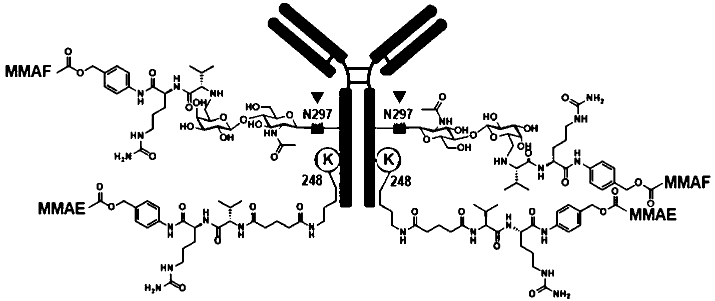 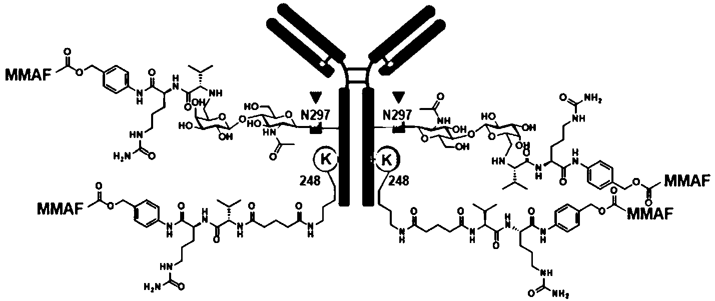 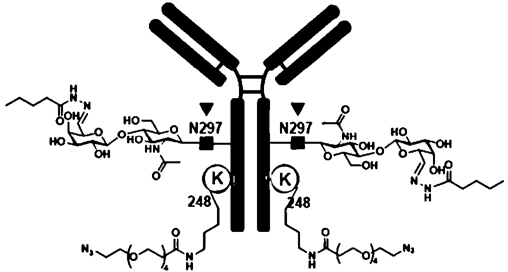 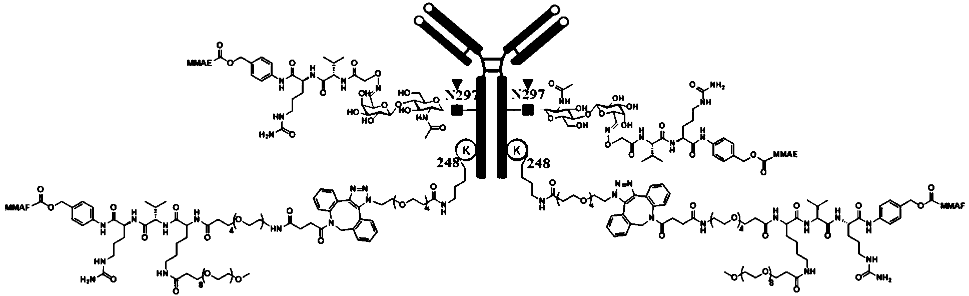 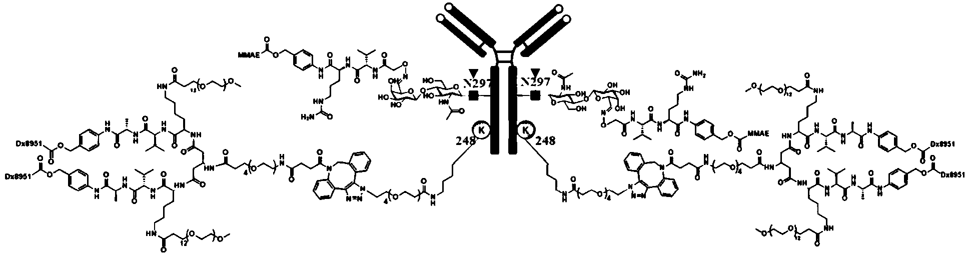 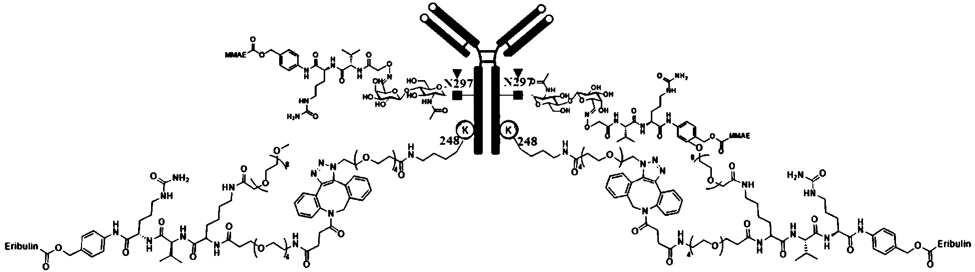 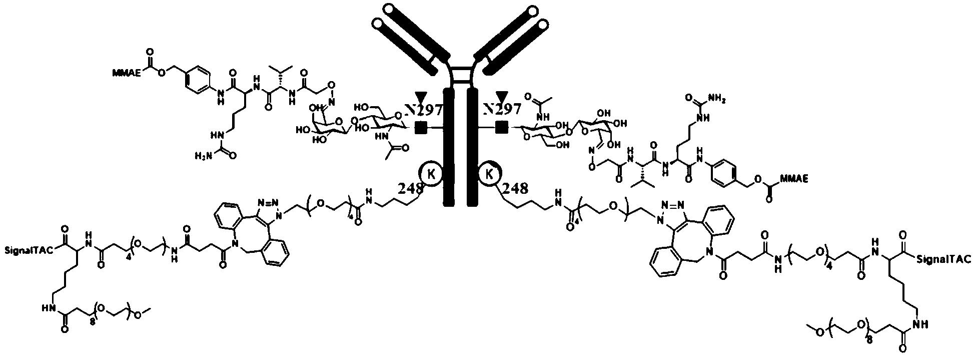 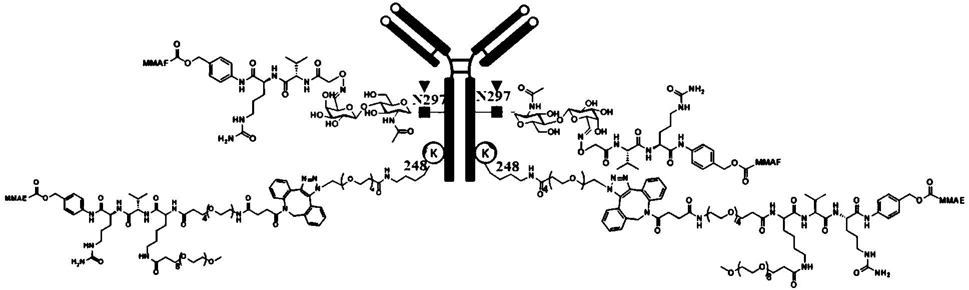 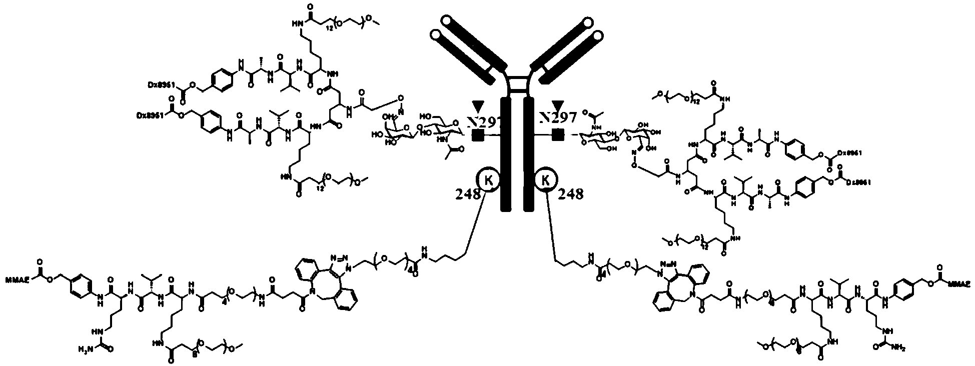 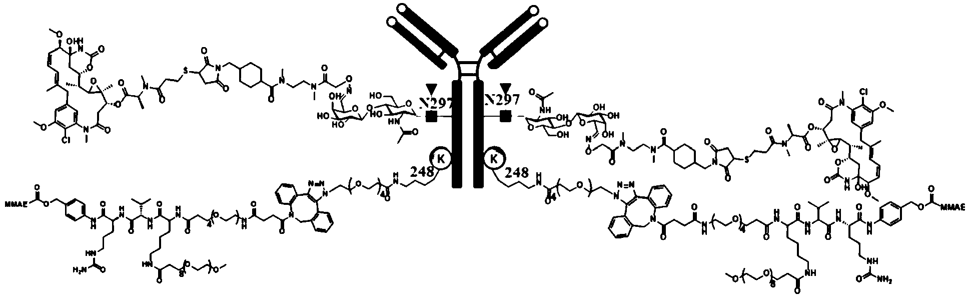 wherein,
MMAE has a structure of 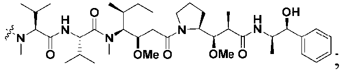
MMAF has a structure of 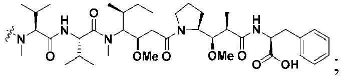
Dx8951 has a structure of 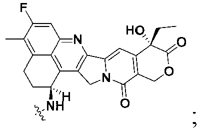
Eribulin has a structure of 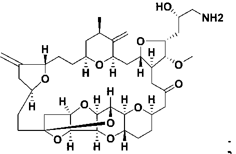
SignalTAC has a structure of

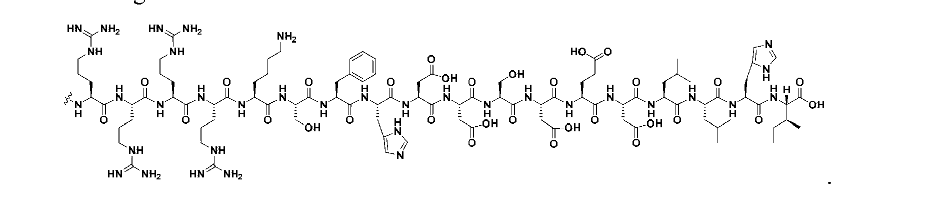

According to one embodiment of the present invention, provided is a method for preparing the above-mentioned dual-site antibody-functional molecule conjugate, which is selected from the group consisting of the following methods I to III, as shown in the following reaction formula: 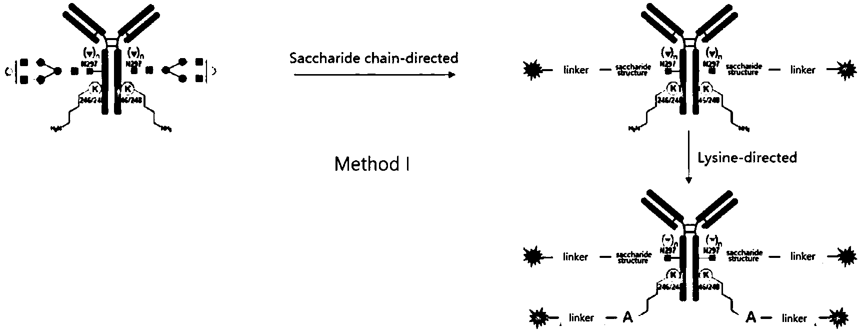 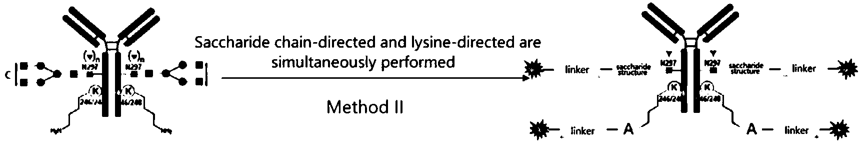 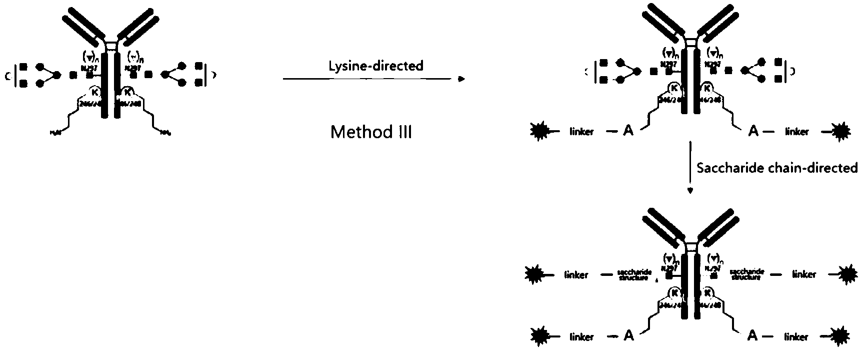 wherein, in the above reaction formula, the definitions of various moieties are as defined above:
Method I:
   subjecting a wild-type antibody or a defucosylating antibody to a saccharide chain-directed technique mediated by a glycosyltransferase or endoglycosidase to obtain a saccharide chain-directed antibody-functional molecule conjugate with a functional molecule modified at the glycosylation site of the antibody,
   subsequently, conjugating a functional molecule-linker to the lysine 246 or 248 of the antibody through a targeting peptide/protein-mediated K246 or K248 site-directed technique to finally obtain a dual-site modified antibody-functional molecule conjugate;
Method II:
   to a wild-type antibody or defucosylating antibody, adding an enzyme and a substrate required for saccharide chain-directed technique, and a substrate required for targeting peptide/protein-mediated lysine-directed technique at the same time, and incubating to obtain a dual-site modified antibody-functional molecule conjugate;
Method III:
   first subjecting a wild-type antibody or a defucosylating antibody to a targeting peptide/protein-mediated K246 or K248 site-directed technique to conjugate a functional molecule-linker at the lysine 246 or 248 of the antibody. Subsequently, using a glycosyltransferase or endoglycosidase-mediated saccharide chain-directed technique to conjugate a functional molecule at the glycosylation site of the antibody to finally obtain a dual-site modified antibody-functional molecule conjugate.
   wherein, the glycosyltransferase comprises, but is not limited to fucosyltransferase, galactosyltransferase, sialyltransferase, etc., and the endoglycosidase includes, but is not limited to Endo-S, Endo-S2, Endo-F3, Endo-M and mutants of these enzymes, and includes fusion proteins formed by fusing the functional regions of these different endoglycosidases.

According to one embodiment of the present invention, the Method I includes:
Step a1: placing an antibody in a buffer solution (e.g., pH 5.5-8.0), adding a saccharide structure-functional molecule complex and a glycosyltransferase or endoglycosidase, and reacting at a temperature of 15°C-37°C for 30 minutes to overnight (e.g., 20 hours) to obtain a saccharide chain-directed antibody-functional molecule conjugate with a functional molecule modified at the glycosylation site of the antibody,
Step a2: then placing the product obtained in step a1 in a buffer solution (e.g., pH 5.5-8.0), adding a targeting peptide-functional molecule complex, reacting the mixture at a temperature of 0°C-40°C for 30 minutes to overnight (e.g., 20 hours) to conjugate the functional molecule-linker at the lysine 246 or 248 of the antibody, finally obtaining a dual-site modified antibody-functional molecule conjugate.

According to one embodiment of the present invention, the method II includes:
placing an antibody in a buffer solution (pH 5.5-8.0), adding a glycosyltransferase or endoglycosidase and a saccharide structure-functional molecule complex, and simultaneously adding a targeting peptide-functional molecule complex, reacting at a temperature of 15°C-37°C for 30 minutes to overnight (e.g., 20 hours), and obtaining a dual-site modified antibody-functional molecule conjugate.

According to one embodiment of the present invention, the method III includes:
Step c1: placing an antibody in a buffer solution (pH 5.5-8.0), adding a targeting peptide-functional molecule complex, and reacting at a temperature of 0°C-40°C for 30 minutes to overnight (e.g., 20 hours) to conjugate the functional molecule-linker at the lysine 246 or 248 of the antibody,
Step c2: then placing the product obtained in step c1 in a buffer solution (pH 5.5-8.0), adding a glycosyltransferase or endoglycosidase and a saccharide structure-functional molecule complex, reacting at a temperature of 15°C-37°C for 30 minutes to overnight (e.g., 20 hours) to finally obtain a dual-site modified antibody-functional molecule conjugate.

The glycosyltransferases described in methods I, II, and III above include, but are not limited to fucosyltransferases, galactosyltransferases, sialyltransferases, and the like. The endoglycosidases include, but are not limited to Endo-S, Endo-S2, Endo-F3, Endo-M, and mutants of these enzymes, and include fusion proteins formed by fusing the functional regions of these different endoglycosidases.

According to one embodiment of the present invention, the intermediate saccharide chain-directed antibody-functional molecule conjugate obtained in step a1 of Method I is directly subjected to the next lysine site-directed modification without purification or is subjected to the next step after purification, and/or
the intermediate K246 or K248 site-directed antibody-functional molecule conjugate obtained in step c1 of method III is directly subjected to the next step of glycosylation site-directed modification without purification or is subjected to the next step after purification.

According to one embodiment of the present invention, the dual-site antibody functional molecule conjugate is prepared by method I or method III, wherein in step a1 of method I and step c2 of method III, a endoglycosidase-mediated saccharide chain site-directed technique is used; in step a2 of method I and step c1 of method III, an Fc-binding peptide is used as the Fc domain-directed fragment of the antibody, and a thioester structure is used as an acyl transfer fragment, specifically:
Method I: subjecting a wild-type or defucosylated antibody to a endoglycosidase-mediated saccharide chain-directed technique to obtain a saccharide chain-directed antibody-functional molecule conjugate with a functional molecule modified at the glycosylation site of the antibody, subsequently conjugating a functional molecule-linker to the lysine 246 or 248 of the antibody using a targeting peptide-mediated K246 or K248 site-directed technique, finally obtaining a dual-site modified antibody-functional molecule conjugate, in which the intermediate saccharide chain-directed antibody-functional molecule conjugate is purified or directly reacted in a reaction system for the next lysine site-directed modification,
Method III: firstly, conjugating a wild-type or defucosylating antibody to a functional molecule-linker at the lysine 246 or 248 of the antibody using a targeting peptide-mediated K246 or K248 site-directed technique, subsequently conjugating a functional molecule at the glycosylation site of the antibody using an endoglycosidase-mediated saccharide chain-directed technique, finally obtaining a dual-site modified antibody-functional molecule conjugate, in which the intermediate K246 or K248 site-directed antibody-functional molecule conjugate is purified or directly reacted in a reaction system for the next step of the glycosylation site-directed modification.

According to one embodiment of the present invention, provided is a method for preparing a dual-site antibody-functional molecule conjugate, which is selected from the group consisting of method IV and method V, as shown in the following reaction formula:
Method IV: 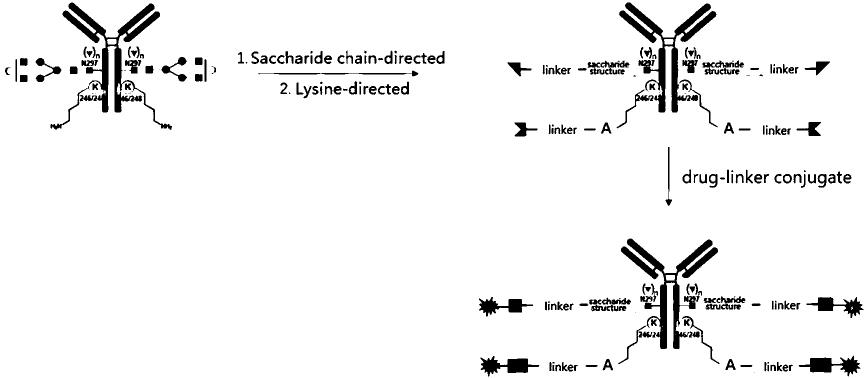
Method V: 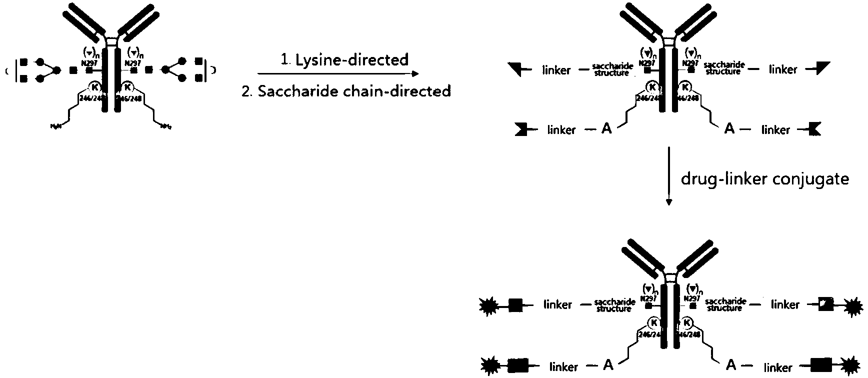 in the above reaction formula, the definitions of various moieties are the same as those in claim 5, and " 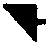 " and " 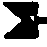 " represent reactive functional groups:
Method IV: subjecting a wild-type antibody or a defucosylating antibody to a glycosyltransferase- or endoglycosidase-mediated saccharide chain-directed technique to obtain a saccharide chain-directed antibody-reactive functional group conjugate with a functional molecule modified at the glycosylation site of the antibody, subsequently conjugating a reactive functional group-linker to the lysine 246 or 248 of the antibody through a targeting peptide- or targeting protein-mediated K246 or K248 site-directed technique to obtain a dual-site modified bifunctional antibody, subsequently introducing functional molecules (such as toxins) into the glycosylation site and the K246 or K248 site through a bioorthogonal reaction, finally obtaining a dual-site modified antibody-functional molecule conjugate;
Method V: First conjugating a wild-type antibody or a defucosylating antibody to a reactive functional group-linker at the lysine 246 or 248 of the antibody through a targeting peptide- or targeting protein-mediated K246 or K248 site-directed technique, subsequently conjugating a reactive functional group to the glycosylation site of the antibody through a glycosyltransferase or endoglycosidase- mediated saccharide chain-directed technique to obtain a dual-site modified bifunctional antibody, subsequently introducing functional molecules (such as toxins) at the glycosylation site and the K246 or K248 site respectively through a bioorthogonal reaction to finally obtain a dual-site modified antibody-functional molecule conjugate,
   wherein, the glycosyltransferase is selected from the group consisting of fucosyltransferase, galactosyltransferase, and sialyltransferase; the endoglycosidase is selected from the group consisting of Endo-S, Endo-S2, Endo-F3, Endo-M, and mutants of these enzymes, and comprises a fusion protein formed by fusing the functional regions of these different endoglycosidases; and the reactive functional group is defined as described above.

According to one embodiment of the present invention, the method IV comprises:
Step b1: placing an antibody in a buffer solution, adding a saccharide structure-reactive functional group complex and glycosyltransferase or endoglycosidase, and reacting at a temperature of 15°C-37°C for 30 minutes to 20 hours to obtain a saccharide chain-directed antibody-reactive functional group conjugate with a reactive functional group modified at the glycosylation site of the antibody; then adding a targeting peptide-reactive functional group complex to the above system and reacting at a temperature of 0°C-40°C for 30 minutes to 20 hours to conjugate the reactive functional group-linker at the lysine 246 or 248 of the antibody to obtain a dual-site modified bifunctional antibody,
Step b2: subsequently adding two functional molecules-linkers to the buffer system of step b1, and reacting at a temperature of 0°C-40°C for 30 minutes to 20 hours to introduce different or same functional molecules at the glycosylation site and the lysine 246 or 248 of the antibody, finally obtaining a dual-site modified antibody-functional molecule conjugate;
   the method V comprises:
   Step d1: placing an antibody in a buffer solution, adding a targeting peptide-reactive functional group complex, and reacting at a temperature of 0°C-40°C for 30 minutes to 20 hours to obtain a lysine-site-directed antibody-reactive functional group conjugate with a reactive functional group modified at the lysine 246 or 248 of the antibody; then adding a saccharide structure-reactive functional group complex and a glycosyltransferase or endoglycosidase to the above system, and reacting at a temperature of 15°C-37°C for 30 minutes to 20 hours to conjugate the reactive functional group-linker at the glycosylation site of the antibody to obtain a dual-site modified bifunctional antibody,
   Step d2: then adding two functional molecule-linkers to the buffer system of step d1 and reacting at a temperature of 0°C-40°C for 30 minutes to 20 hours to introduce different or same functional molecules at the glycosylation site and the lysine 246 or 248 of the antibody, finally obtaining a dual-site modified antibody-functional molecule conjugate.

According to one embodiment of the present invention,
the intermediate saccharide chain-directed antibody-reactive functional group conjugate and the dual-site modified bifunctional antibody obtained in step b1 of method IV can be directly processed into the next step without purification or after ultrafiltration purification, and/or
the intermediate K246 or K248 site-directed-reactive functional group conjugate and the dual-site modified bifunctional antibody obtained in step d1 of method V can be directly used for the next step without purification or after ultrafiltration purification.

According to one embodiment of the present invention, the method is carried out by the following method VI.

### Method VI:

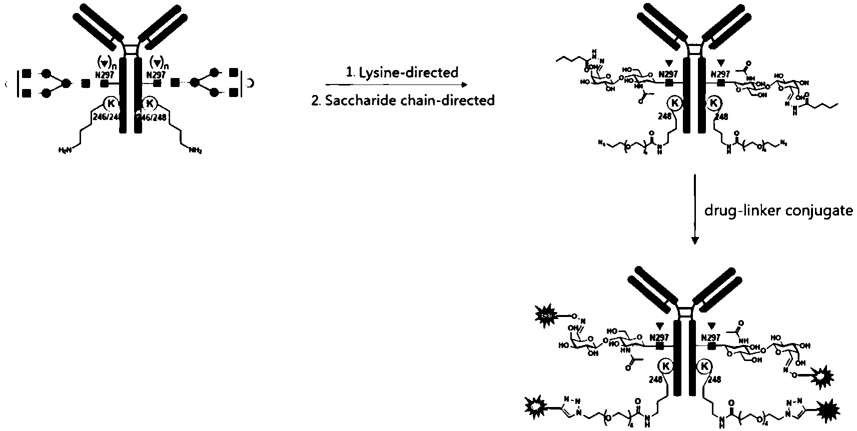

in the above reaction formula, the definitions of various moieties are the same as those in the previous text,
Method VI: subjecting a wild-type antibody or a defucosylated antibody to the saccharide chain-directed technique mediated by endoglycosidase to obtain a saccharide chain-directed antibody-reactive functional group conjugate with an acylhydrazone structure modified at the glycosylation site of the antibody, subsequently conjugating an azide functional group to the lysine 246 or 248 of the antibody through a targeting peptide- or targeting protein-mediatedK246 or K248 site-directed technique to obtain a bifunctional antibody with dual-site modification of acylhydrazone and azide; then introducing functional molecules at the glycosylation site and the K246 or K248 site respectively through acylhydrazone-oxime exchange chemistry and click chemistry reactions to finally obtain a dual-site modified antibody-functional molecule conjugate.

According to one embodiment of the present invention, the method VI comprises:
Step e1: Placing an antibody in a pH 6.0-8.0 buffer, adding a saccharide structure-reactive functional group complex and glycosyltransferase or endoglycosidase, and reacting at a temperature of 15°C-37°C for 30 minutes to 20 hours to obtain a saccharide chain-directed antibody-reactive functional group conjugate with a functional molecule modified at the glycosylation site of the antibody; then adding a targeting peptide-reactive functional group complex to the above system, using different concentrations of organic solvents to assist solubilization, and reacting at a temperature of 0°C-40°C for 30 minutes to 20 hours to conjugate the reactive functional group-linker at the lysine 246 or 248 of the antibody to obtain a dual-site modified bifunctional antibody,
Step e2: then adding two functional molecule-linkers and a catalyst for catalyzing acylhydrazone-oxime exchange chemistry to the buffer system of step e1, and reacting at a temperature of 0°C-40°C for 30 minutes to 20 hours to introduce different or same functional molecules at the glycosylation site and the lysine 246 or 248 of the antibody, finally obtaining a dual-site modified antibody-functional molecule conjugate.

The endoglycosidases in the above method include, but are not limited to Endo-S, Endo-S2, Endo-F3, Endo-M and mutants of these enzymes, and include fusion proteins formed by fusing the functional regions of these different endoglycosidases.

In another aspect, the present invention provides the use of the above-mentioned dual-site antibody-functional molecule conjugate in preparation of a drug, a diagnostic and therapeutic reagent, a kit, etc., wherein the drug, diagnostic and therapeutic reagent, and kit are used to treat a tumor, a disease caused by inflammation, a disease caused by viral infection, or an immune disease.

In one embodiment of the present invention, the tumor is selected from the group consisting of ovarian cancer, breast cancer, fallopian tube cancer, endometrial cancer, peritoneal cancer, gastric cancer, colon cancer, bladder cancer, pancreatic cancer, biliary tract cancer, osteosarcoma, cervical cancer, head and neck tumors, germ cell and embryonal cancer, esophageal cancer, malignant glioma, Ewing sarcoma, melanoma, bile duct cancer, prostate cancer, small cell lung cancer, non-small cell lung cancer, lymphoma and blood cancer;
the disease caused by inflammation is selected from the group consisting of conjunctivitis, bronchitis, sinusitis, Crohn's disease, etc.;
the disease caused by the viral infection is selected from the group consisting of influenza, herpes zoster, human papillomavirus infection, AIDS, hepatitis A, hepatitis B, chickenpox, etc.;
the immune disease is selected from the group consisting of rheumatoid arthritis, systemic lupus erythematosus, myasthenia gravis, myeloma, dermatomyositis, psoriasis, etc.

### Beneficial effects

The present invention combines saccharide chain-directed technique with targeting peptide/protein-mediated K246 or K248 site-directed technique to achieve the introduction of functional molecules at both the glycosylation site and the lysine 246 or 248 of the antibody, which is useful for preparing antibody-functional molecule conjugates, fluorescent diagnostic reagents, etc. The prepared dual-site antibody-functional molecule conjugates have excellent stability, cytotoxicity and in vivo activity with good drugability.

The specific dual sites selected in the present invention are the glycosylation site and the K246 or K248 site. Compared with the technologies used in other dual-site-directed antibody-functional molecule conjugates, the site-specific technique used to prepare the dual-site-directed antibody-functional molecule conjugates of the present invention does not require in vivo engineering of the antibody, is applicable to various types of antibodies, and has the advantages of simple operation and fewer purification steps with good drugability and easy industrialization. Compared with other single-site conjugates with high DAR values or dual-drug antibody-functional molecule conjugates, the dual site technique of the present invention can disperse the local hydrophobicity of the antibody-functional molecule conjugates and avoid excessive local space congestion, thereby increasing stability.

### BRIEF DESCRIPTION OF THE DRAWINGS

Figs. 1-2 show the aggregation stability results of the dual-site directed antibody-functional molecule conjugates of the present application.
Figs. 3-4 show the homogeneity and hydrophilicity results of the dual-site directed antibody-functional molecule conjugates of the present application. The ADC compounds prepared by the method of the present application showed good homogeneity.
Figs. 5-7 show the results of in vitro activity assays of the dual-site directed antibody-functional molecule conjugates of the present application. Fig. 5 shows the inhibition rates of these dual-site antibody-functional molecule conjugates on Her2-positive SK-Br-3 cells; Fig. 6 shows the inhibition rates on Her2-positive NCI-N87 cells; and Fig. 7 shows the effects on the survival rate of Her2-negative MDA-MB-231 cells.
Fig. 8 shows the results of in vitro activity assays of the dual-site directed antibody-functional molecule conjugates of the present application. Compared with single-drug ADC compounds, some dual-site antibody-functional molecule conjugates showed better inhibition rates against high and medium Trop2 expressing cells.
Figs. 9-10 show theresults of in vivo anti-tumor activity assays of the dual-site directed antibody-functional molecule conjugates of the present application, which respectively demonstrate the inhibition of the dual-site antibody-functional molecule
conjugates on the tumor volume in vivo and the effect on the body weight of nude mice.

### DETAILED DESCRIPTION

The glycosidases used in this invention were expressed in an Escherichia coli system and were laboratory-produced. The small molecule cytotoxic drugs MMAE and MMAF used in this invention were purchased from Shanghai RESUPERPHARMTECH Co., Ltd.; the Fc-binding peptide was custom-made from Nanjing GenScript Biotech Co., Ltd.; protein A was purchased from Sangon Biotech (Shanghai) Co., Ltd.; and 2-(Triacylthio) acetic acid was purchased from Shanghai Bide Pharmaceutical Technology Co., Ltd. All other compounds and reagents, unless otherwise specified, were purchased from Sinopharm Chemical Reagent Co., Ltd.

The instruments and chromatographic columns used in the present invention include: Waters Xevo G2-XS QTOF, analytical high performance liquid chromatograph (Thermo ultimate 3000), analytical high performance liquid chromatograph (Beijing Chuangxintongheng LC3000), preparative high performance liquid chromatograph (Beijing Chuangxintongheng LC3000), C18 analytical column (Thermo, AcclaimTM 120, 5 µm, 4.6 x 250 mm), C18 analytical column (Agilent, SB-C18, 4.6 x 150 mm), mass spectrometry C18 column (Waters, ACQUITY UPLC BEH C18, 1.7 mm, 2.1 x 50 mm).

Instrument for determining molecular weight of Antibody: Liquid chromatography-mass spectrometry (LC-MS), Waters Xevo G2-XS Q-TOF, equipped with a C4 column (ACQUITY UPLC Protein BEH C4, 1.7µm, 2.1mm x 50mm).

### General operation I:

### Endoglycosidase-based one-step method for preparing a saccharide chain-directed antibody-functional molecule conjugate

The prepared disaccharide-toxin complex (compounds 2a or 2b), a wild-type antibody, and the wild-type endoglycosidase Endo-S2 were added at 0.5 mM, 5 mg/mL, and 0.4 mg/mL, respectively. The reaction system was adjusted to pH 7.0 and incubated at 30°C for 0.5 h to 12 h. After LC-MS confirmation of conversion to product, the desired saccharide chain-directed and quantitative antibody-functional molecule conjugates GsADC-3a to GsADC-3b were obtained by protein A purification, as shown in Examples 16-17 below.

### General operation II:

### Targeting peptide-based one-step method for preparing K248 site-directed antibody-functional molecule conjugates

The prepared affinity peptide-toxin complexe (compounds 4a or 4b) and a wild-type antibody were added at 0.5 mM and 5 mg/mL, respectively, and a PB buffer solution (50 mM, pH 7.4, containing 20% DMF) was added. The reaction system was adjusted to pH 7.4 and incubated at 37°C for 2 h to 4 h. After LC-MS confirmation of conversion to product, the desired K248 site-directed and quantitative antibody-functional molecule conjugates KsADC-**5a** to KsADC-5**b** were obtained by protein A purification, as shown in Examples **22-23** below.

### General operation III:

### Preparation method of dual-site antibody-functional molecule conjugates by combining saccharide chain-directed technique and affinity peptide-directed modification technique

The prepared disaccharide-toxin complex (compounds **2a** or **2b),** a wild-type antibody, and the wild-type endoglycosidase Endo-S2 were added at 0.5 mM, 5 mg/mL, and 0.4 mg/mL, respectively. The reaction system was adjusted to pH 7.0 and incubated at 30°C for 0.5 h to 12 h. After LC-MS confirmed the conversion to a uniform saccharide chain-directed antibody functional molecule conjugate, the prepared affinity peptide-toxin complex (compounds **4a** or **4b)** was added to the reaction system at 0.5 mM. The resultant was incubated at 37°C for 2 h to 4 h. After LC-MS confirmed the conversion to product, the dual-site antibody-functional molecule conjugates ADC-**6a** to ADC-**6d** were obtained by protein A purification, as shown in Examples **30-33** below.

### General operation IV:

### Preparation method of dual-site antibody-functional molecule conjugates by combining saccharide chain-directed technique and affinity peptide-directed modification technique

The prepared affinity peptide-toxin complex (compound **4a)** and a wild-type antibody were added at 0.5 mM and 5 mg/mL, respectively. The reaction system was adjusted to neutrality and incubated at 37°C for 2 h to 4 h. After LC-MS confirmed conversion to a uniform K248-directed antibody-functional molecule conjugate, the prepared disaccharide-toxin complex (compound **2a)** and the wild-type endoglycosidase Endo-S2 were added to the reaction system at concentrations of 0.5 mM and 0.4 mg/mL, respectively. The reaction system was incubated at 30°C for 0.5 h to 12 h. After LC-MS confirmed conversion to product, the two-site antibody-functional molecule conjugate **ADC-6a-1** was obtained by protein A purification, as shown in Example **34** below.

### General operation V:

### Preparation method of dual-site antibody-functional molecule conjugates by combining saccharide chain-directed technique and affinity peptide-directed modification technique

The prepared disaccharide-toxin complex (compound **2a),** the affinity peptide-toxin complex (compound **4a),** a wild-type antibody, and the wild-type endoglycosidase Endo-S2 were added at 0.5 mM, 0.5 mM, 5 mg/mL, and 0.4 mg/mL, respectively. The reaction system was adjusted to neutral and incubated at 30°C for 0.5 h to 12 h. After LC-MS confirmation of conversion to product, the two-site antibody-functional molecule conjugate ADC-**6a-2** was obtained by protein A purification, as shown in Example **35** below.

### General operation VI:

### Preparation method of dual-site antibody-functional molecule conjugates by combining saccharide chain-directed technique and affinity peptide-directed modification technique

Step 1: The prepared disaccharide-functional molecule complex (compound **2c),** a wild-type antibody, and the wild-type endoglycosidase Endo S2 were dissolved in a 50 mM His-HCl buffer system at pH 6.5 to 0.5 mM, 5 mg/mL, and 0.1 mg/mL, respectively. The reaction system was adjusted to pH 7.0 and incubated at 30°C for 1 h. After LC-MS confirmed conversion to a uniform saccharide chain-directed antibody-functional molecule conjugate, the buffer system was replaced with a 50 mM PIPES at pH 7.4 by ultrafiltration concentration. The affinity peptide-functional molecule complex (compound **4c)** was added to the system to 0.34 mM. 20% DMF was added for solubilization. The reaction system was incubated at 37°C for 10 h. After LC-MS confirmed conversion to product, the reaction system was concentrated by ultrafiltration to obtain bifunctional antibodies **Ab-1** to **Ab-2,** as shown in Examples **28-29** below.

Step 2: The bifunctional antibodies prepared above, a drug-linker **D1-D3/D9,** a drug-linker **D4-D8,** and the catalyst m-aminobenzoic acid were added to a His-HCl buffer (50 mM, pH 6.0, containing 20% DMF) to 5 mg/mL, 0.34 mM, and 0.16 mM, respectively. The mixture was incubated at 37°C for 8 h. LC-MS confirmed the conversion into homogeneous dual-site antibody-functional molecule conjugates **ADC-7 to ADC-19,** as shown in Examples **36-48** below.

In general operation VI, the catalyst may be a catalyst for acylhydrazone-oxime exchange reaction, such as m-aminobenzoic acid or other aniline catalysts.

### General Operation VII:

### Preparation method of single-drug ADCs by combining saccharide chain-directed technique and acylhydrazone-oxime exchange chemistry

The prepared disaccharide-functional molecule complex (compound **2c),** a wild-type antibody, and the wild-type endoglycosidase Endo S2 were dissolved in a 50 mM His-HCl buffer system at pH 6.5 to 0.5 mM, 5 mg/mL, and 0.1 mg/mL, respectively. The reaction system was adjusted to pH 7.0 and incubated at 30°C for 1 h. LC-MS confirmed the conversion into a uniform saccharide chain-directed antibody-functional molecule conjugate. After ultrafiltration concentration, the homogeneous saccharide chain-directed antibody-functional molecule conjugate and a drug-linker **D1** or **D9** were dissolved in a His-HCl buffer system (50 mM, pH 6.0, containing 136 mM m-aminobenzoic acid and 20% DMF) to 5 mg/mL and 0.34 mM, respectively. The mixture was incubated at 30°C for 4-8 hours. After LC-MS confirmation of conversion to a homogeneous N297 site antibody-drug conjugate, **GsADC-3c to GsADC-3f** were obtained by protein A purification, as shown in Examples **18** to **21** below.

### General operation VIII:

### Preparation method of single-drug ADCs by combining K-site-directed technique and click chemistry

The prepared affinity peptide-functional molecule complex (compound **4c)** and a wild-type antibody were dissolved in a 50 mM PIPES buffer at pH 7.4 at 0.34 mM and 5 mg/mL, respectively. The mixture was incubated at 37°C for 10 h, and LC-MS confirmed conversion to a homogeneous K-site antibody-functional molecule conjugate. After ultrafiltration concentration, the homogeneous antibody-functional molecule conjugate and a drug-linker **D5** or **D7** were dissolved in a 50 mM PB buffer at pH 7.4 at 5 mg/mL and 0.17 mM, respectively. The mixture was incubated at 30°C for 4 h. After LC-MS confirmed conversion to a homogeneous K248-site antibody-drug conjugate, **KsADC-5c to KsADC-5f** were obtained by Protein A purification, as shown in Examples **24-27** below.

I: Preparation of disaccharide-functional molecule complexes (such as disaccharide-toxin complexes)

### Example 1-2: Synthesis of Compounds 2a and 2b

The structures and synthesis methods of compounds **2a** and **2b** are as follows:

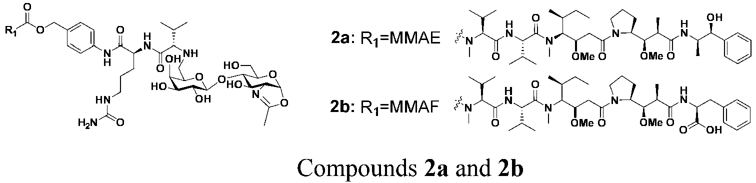

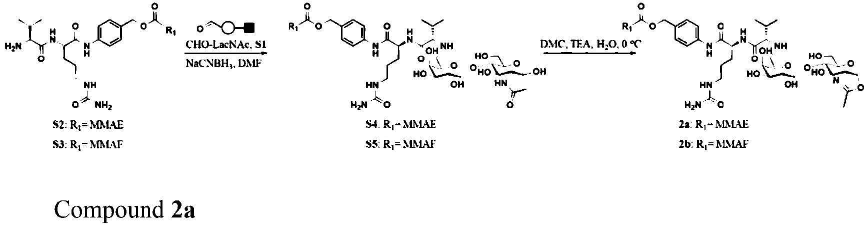

Step 1: **S2** (11 mg, 9.8 mM) was dissolved in 500 mL of DMF. CHO-LacNAc (S1, 14.8 mg, 38.8 mM) was added to the reaction system. DMF was then added to a DMF/0.2M PB ratio of 1:1. The reaction system was adjusted to pH 6.0 with NaOH/HCl, followed by the addition of sodium cyanoborohydride (6.3 mg, 100 mM). The reaction was conducted at room temperature for 2 h. LC-MS indicated the reaction was nearly complete. The product was separated and purified using a semi-preparative C18 column. The target fraction was collected and lyophilized to yield **S4** (81% yield). HRMS: Calcd. for C₇₂H₁₁₇N₁₁O₂₂: [M+2H]²⁺ 744.9187, found: 744.9110.

Step 2: **S4** (20 mg, 10 mM) was dissolved in 400 µL of DMF/H₂O (1:1). DMC (67.6 mg, 300 mM) and triethylamine (120 mg, 900 mM) were added to the reaction system, mixed thoroughly, and then cooled to 0°C on ice and reacted for 2 h. LC-MS indicated the reaction was nearly complete. The resultant was separated and purified using a basic C18 column, and the target product was collected and lyophilized to obtain **2a** (87 % yield). HRMS Calcd. for C₇₂H₁₁₅N₁₁O₂₁: [M+2H]²⁺ 735.9134, found: 735.8130.

### Compound 2b

Step 1: **S3** (11 mg, 9.7 mM) was dissolved in 500 mL of DMF. CHO-LacNAc **(S1,** 14.8 mg, 38.8 mM) was added to the reaction system. DMF was then added to a DMF/0.2 M PB ratio of 1:1. The reaction system was adjusted to pH 6.0 with NaOH/HCl, followed by the addition of sodium cyanoborohydride (6.3 mg, 100 mM). The reaction was conducted at room temperature for 2 h. LC-MS confirmed the reaction was nearly complete. The product was separated and purified using a semi-preparative C18 column. The target fraction was collected and lyophilized to yield **S5** (75% yield). HRMS: Calcd. for C₇₂H₁₁₃N₁₁O₂₁: [M+2H]²⁺ 751.9083, found: 751.9417.

Step 2: **S5** (20 mg, 10 mM) was dissolved in 400 mL of DMF/H₂O (1:1). DMC (67.6 mg, 300 mM) and triethylamine (120 mg, 900 mM) were added to the reaction system, mixed thoroughly, and then cooled to 0°C on ice and reacted for 2 h. LC-MS indicated the reaction was nearly complete. The resultant was separated and purified using a basic C18 column, and the target product was collected and lyophilized to obtain **2b** (88 % yield). HRMS Calcd. for C₇₂H₁₁₃N₁₁O₂₂: [M+2H]²⁺ 742.9031, found: 742.9319.

### Example 3: Synthesis of Compound 2c

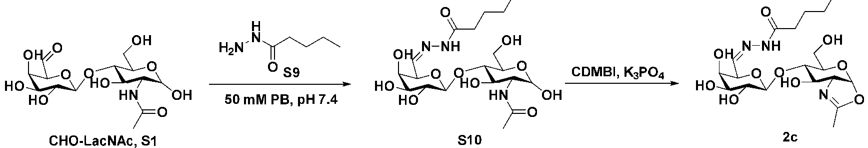

Step 1: CHO-LacNAc **(S1,** 20 mg, 52.5 µmol) was dissolved in 1 mL of a 50 mM phosphate buffer at pH **7.4. S9** (15.24 mg, 131.3 µmol) was added to the reaction system and reacted at 30°C for 4 h. LC-MS indicated the reaction was nearly complete. The target fraction was isolated and purified using a semi-preparative column, and lyophilized to yield **S10** (90% yield). HRMS Calcd. for C₁₄H₃₃N₃O₁₁[M+H]⁺ 480.2193, found: 480.2424.

Step 2: **S10** (20 mg, 41.8 µmol) was dissolved in 2 mL of 50 mM PB buffer at pH 8.0. 2-chloro-1,3-dimethyl-1H-benzimidazol-3-ium chloride (136 mg, 0.627 mmol) and potassium phosphate (265 mg, 1.25 mmol) were added to the above reaction system. The mixture was then cooled to 0°C on ice and reacted for 2 h. LC-MS indicated that the reaction was nearly complete. The target product was separated and purified using a basic column, and collected and lyophilized to obtain **2c** (87% yield). HRMS Calcd. for C₁₉H₃₁N₃O₁₀[M+H]⁺: 462.2088, found: 462.1957.

### II: Preparation of affinity peptide-functional molecule complexes (such as affinity peptide-toxin complexes)

### Example 4-5: Synthesis of Compounds 4a and 4b

The structures and synthesis methods of compounds **4a** and **4b** are as follows:

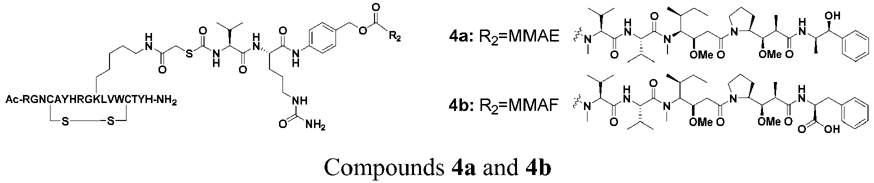

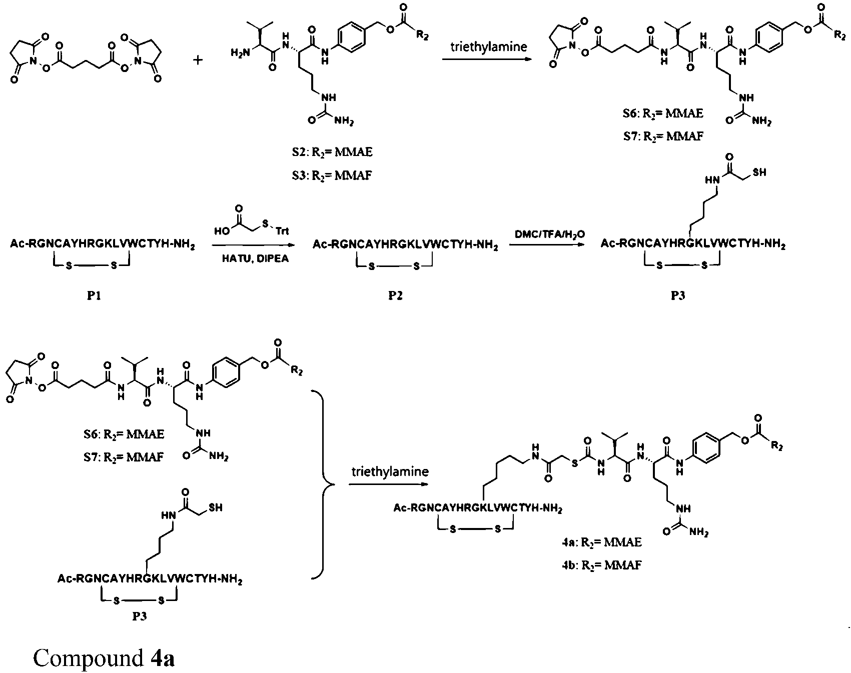

Step 1: **S2** (10 mg, 9.8 µmol) was weighed and dissolved in DMF (500 µL). Disuccinimidyl glutarate (DSG, 15.6 mg, 0.048 mM) and triethylamine (4 µL, 0.029 mM) were added. The mixture was reacted at room temperature for 2 h. LC-MS indicated that the reaction was complete, the product was isolated and purified using semi-preparative column and lyophilized to obtain **S6** as a white powder (yield 91 %). HRMS, Calcd. for C₆₇H₁₀₃N₁₁O₁₇: [M+H]⁺ 1334.7567, found: 1334.7655.

Step 2: **S8** (15.7 mg, 0.047 mM) was weighed and dissolved in DMF. HATU (7.14 mg, 0.0188 mM), DIPEA (4.84 µL, 0.028 mM), and **P1** (20 mg, 0.0094 mM) were added in sequence. The mixture was stirred at room temperature for 1 h. LC-MS indicated that the reaction was complete, the product was isolated and purified using semi-preparative column and lyophilized to obtain **P2** as a white powder(yield 92%). HRMS, Calcd. for C₁₁₄H₁₅₁N₃₁O₂₃S₃: [M+H]⁺ 2419.0839, [M+3H]³⁺ 807.0332, [M+4H]⁴⁺ 605.5269, found: 807.0342, 605.5232.

Step 3: **P2** (15 mg, 0.0062 mM) was weighed and dissolved in 500 µL of dichloromethane. Trifluoroacetic acid (450 µL) and triisopropylsilane (50 µL) were added in an ice bath. The mixture was reacted at room temperature for 1 h, and then dried under nitrogen to obtain **P3.** HRMS, Calcd. for C₉₅H₁₃₇N₃₁O₂₃S₃: [M+H]⁺ 2176.9744, [M+3H]³⁺ 726.3300, [M+4H]⁴⁺ 544.9995, found: 726.3278, 545.0035.

Step 4: **S6** (13.3 mg, 0.01 mM) was weighed and dissolved in a DMF/PB7.4 (1/1) mixture. **P3** was added and allowed to react at room temperature for 30 min. The product was then isolated and purified using semi-preparative column and lyophilized to obtain **4a** as a white powder, (yield 76%). HRMS, Calcd. for C₁₅₈H₂₃₅N₄₁O₃₇S₃ [M+H]⁺3395.7007, [M+3H]³⁺1132.5721, [M+4H]⁴⁺ 849.6810, found: 1133.5587, 849.6596.

### Compound 4b

Step 1: **S3** (10 mg, 9.8 µmol) was weighed and dissolved in DMF (500 µL). Disuccinimidyl glutarate (DSG, 15.6 mg, 0.048 mM) and triethylamine (4 µL, 0.029 mM) were added. The mixture was reacted at room temperature for 2 h. LC-MS indicated that the reaction was complete, the product was isolated and purified using semi-preparative column and lyophilized to obtain **S7** as a white powder(yield 91 %). HRMS, Calcd. for C₆₈H₁₀₂N₁₀O₁₈: [M+H]⁺ 1346.7374, found: 1346.7935.

Step 2: **S7** (13.3 mg, 0.01 mM) was weighed and dissolved in a 1/1 DMF/PB7.4 mixture. **P3** was added and allowed to react at room temperature for 30 min. The product was then isolated and purified using a semi-preparative column and lyophilized to obtain **4b** as a white powder (yield 76 %). HRMS, Calcd. for C₁₅₉H₂₃₃N₄₀O₃₈S₃: [M+H]⁺3407.6692, [M+3H]³⁺1136.8897, [M+4H]⁴⁺ 852.6692. Found: 1137.9250, 853.6930.

### Example 6: Synthesis of Compound 4c

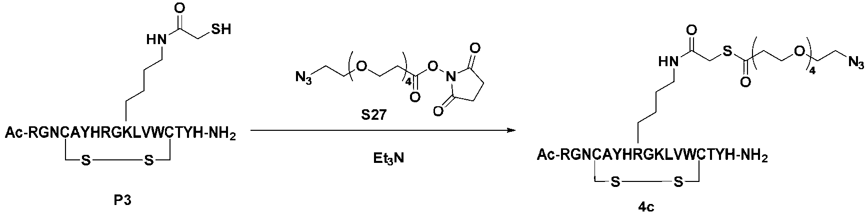

Step 1: **P3** (50 mg, 23 µmol) was weighed and dissolved in 500 µL of DMF. **S27** (13.4 mg, 34.5 µmol) and triethylamine (7 mg, 69 µmol) were added to the above system and mixed. The mixture was reacted at 30°C for 4 h. The product was separated and purified using a semi-preparative column and lyophilized to obtain **4c** (36.6 mg, 65% yield). HRMS: Calcd. for C₁₀₆H₁₅₆N₃₄O₂₈S₃ [M+2H]²⁺ 1225.5573, found: 1225.586.

### III: Synthesis of drug-linkers

### Example 7: Synthesis of Compound D1

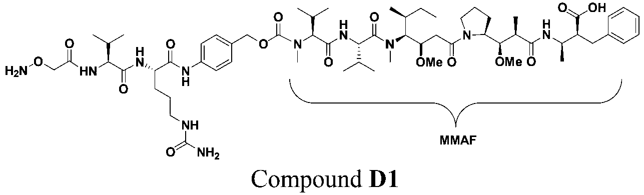

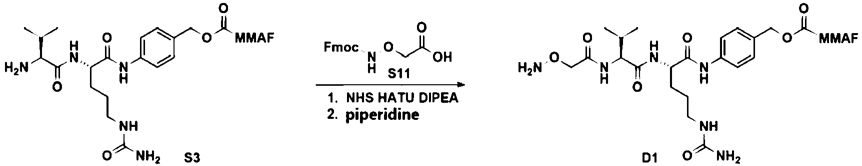

Step 1: **S11** (100 mg, 319.2 µmol) was weighed and dissolved in 1 mL of DMF. NHS (44 mg, 382.6 µmol), HATU (145.8 mg, 382.6 µmol), and DIPEA (123.8 mg, 957.6 µmol) were added in sequence. After reacting at room temperature for 1 h, H₂N-VC-PAB-MMAF **(S3,** 181.3 mg, 159.6 µmol) and DIPEA (41.3 mg, 319.2 µmoL) were added to the system and mixed. The mixture was reacted at 30°C for 2 h. After LC-MS confirmed that the reaction was complete, 0.2 mL of piperidine was added to the reaction system and reacted at room temperature for 20 min. The product was separated and purified using a semi-preparative column and lyophilized to obtain **D1** (146.5 mg, yield 76%). HRMS, Calcd. for C₆₂H₉₉N₁₁O₁₅ [M+H]⁺1238.7400, [M+2H]²⁺ 619.8739, found: 1238.7392, 619.8727.

### Example 8: Synthesis of Compound D2

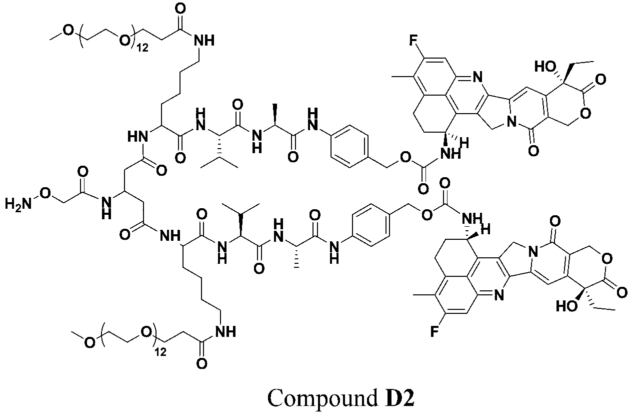

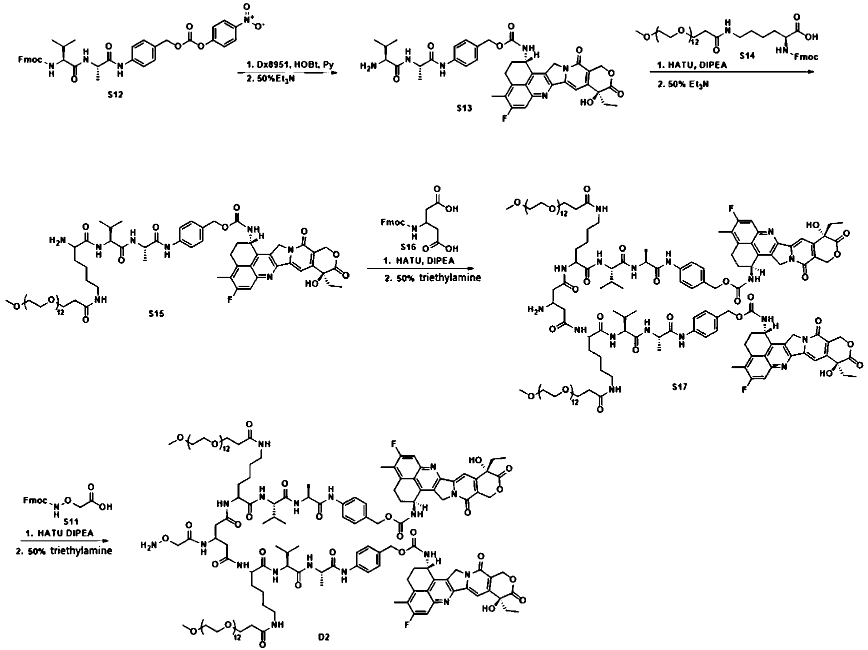

Step 1: Fmoc-VA-PAB-PNP **(S12,** 40 mg, 59 µmol) was weighed and dissolved in 800 µL of DMSO. Dx8951 (31.3 mg, 59 µmol), HOBt (1.6 mg, 12 µmol), and pyridine (370.2 mg, 4.68 mmol) were added to the above reaction system and mixed. The mixture was reacted at 30°C overnight. After LC-MS confirmed the completion of the reaction, an equal volume of triethylamine was added, and the reaction was carried out at 30°C for 2 h. The product was separated and purified using a semi-preparative column and lyophilized to obtain H₂N-VA-PAB-Dx8951 **(S13,** 28.5 mg, 64% yield). HRMS calcd for C₄₀H₄₃FN₆O₈ [M+H]⁺ 755.3204, found: 755.3214.

Step 2: **S14** (65.1 mg, 66.3 µmol) was weighed and dissolved in 650 µL of DMF. HATU (50.4 mg, 132.6 µmol), **S13** (50 mg, 66.3 µmol), and DIPEA (25.7 mg, 198.9 µmol) were added in sequence and mixed. The mixture was reacted at room temperature for 2 h. After LC-MS confirmed the completion of the reaction, an equal volume of triethylamine was added, and the reaction was carried out at 30°C for 2 h. The product was separated and purified using a semi-preparative column and lyophilized to obtain **S15** (74.4 mg, 75% yield). HRMS, calcd for C₇₄H₁₀₉FN₈O₂₃ [M+H]⁺ 1497.7668, [M+2H]²⁺ 749.3873, found: 1497.7222, 749.3452.

Step 3: **S16** (20 mg, 54.2 µmol) was weighed and dissolved in 200 µL of DMF. HATU (61.8 mg, 162.6 µmol), **S15** (202.7 mg, 135.5 µmol), and DIPEA (20 mg, 162.6 µmol) were added in sequence and mixed. The mixture was reacted at 30°C for 2 h. After LC-MS confirmed the completion of the reaction, an equal volume of triethylamine was added and the mixture was reacted at 30 °C for 2 h. The product was separated and purified using a semi-preparative column and lyophilized to obtain **S17** (72.5 mg, 43% yield). HRMS, calcd for C₁₅₃H₂₂₂F₂N₁₇O₄₈ [M+2H]²⁺ 1553.2827, [M+3H]³⁺ 1035.8577, [M+4H]⁴⁺ 777.1452, found: 1553.2460, 1035.8512, 777.1463.

Step 4: **S11** (0.92 mg, 3 µmol) was weighed and dissolved in 200 µL of DMF. HATU (2.3 mg, 6 µmol), **S17** (10 mg, 3.2 µmol) and DIPEA (1.1 mg, 9 µmol) were added in sequence. The mixture was reacted at 30°C for 2 h. After LC-MS confirmed that the reaction was complete, an equal volume of triethylamine was added and the mixture was reacted at 30°C for 2 h. The mixture was separated and purified using a semi-preparative column and lyophilized to obtain **D2** (7.7 mg, 81% yield). HRMS, Calcd. for C₁₅₅H₂₂₆F₂N₁₈O₅₀ [M+2H]²⁺ 1589.7909, [M+3H]³⁺ 1060.1965, [M+4H]⁴⁺ 795.3993, found: 1589.7994, 1060.1973, 795.4008.

### Example 9: Synthesis of Compound D3

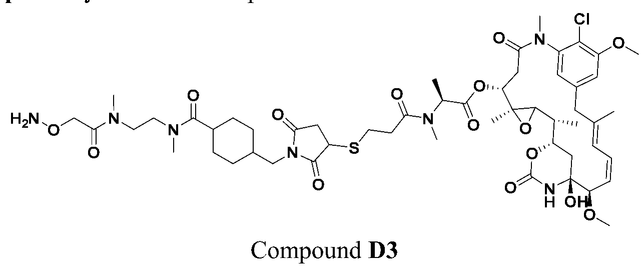

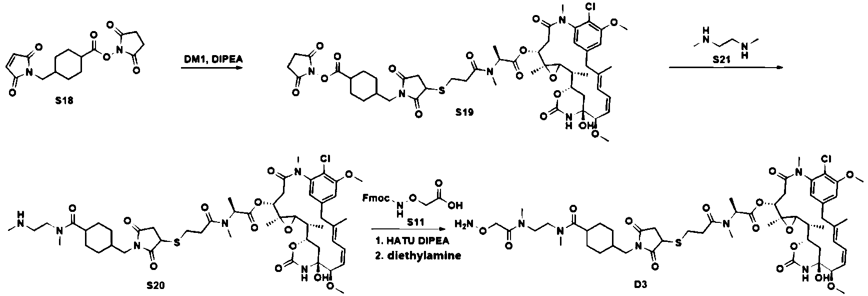

Step 1: **S18** (54.4 mg, 162.8 µmol) was weighed and dissolved in 544 µL of DMF. DM1 (100 mg, 135.7 µmol) and DIPEA (35 mg, 271.4 µmol) were added and reacted at 30°C for 2 h. The product was separated and purified using a semi-preparative column and lyophilized to obtain SMCC-DM1 **(S19,** 125 mg, 86% yield). HRMS: Calcd. for C₅₁H₆₆ClN₅O₁₆S [M+H]⁺ 1072.3992, found: 1071.3914.

Step 2: **S19** (80 mg, 74.7 µmol) was weighed and dissolved in 800 µL of DMF. N,N'-dimethylethylenediamine **(S21,** 65.7 mg, 747 µmol) was added and the reaction was incubated at 30°C for 3 h. **S20** (67 mg, 86% yield) was isolated and purified using a semi-preparative column. HRMS: Calcd. for C₅₁H₇₃ClN₆O₁₃S [M+H]⁺ 1045.4723, found: 1045.4816.

Step 3: **S11** (15 mg, 47.9 µmol) was weighed and dissolved in 150 µL DMF. HATU (54.7 mg, 144 µmol), **S20** (25 mg, 24 µmol) and DIPEA (31 mg, 240 µmol) were added in sequence. The mixture was reacted at 30°C for 2 h. After LC-MS confirmed that the reaction was complete, diethylamine (17.5 mg, 240 µmol) was added and the mixture was reacted at 30°C for 1 h. The product was separated and purified using a semi-preparative column and lyophilized to obtain H₂N-O-MCC-DM1 **(D3,** 18 mg, yield 67%). HRMS, calcd for C₅₃H₇₆ClN₇O₁₃S [M+H]⁺ 1118.4887, found: 1118.4950.

### Example 10: Synthesis of Compound D4

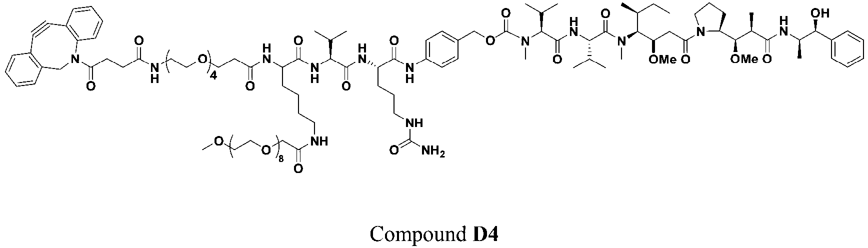

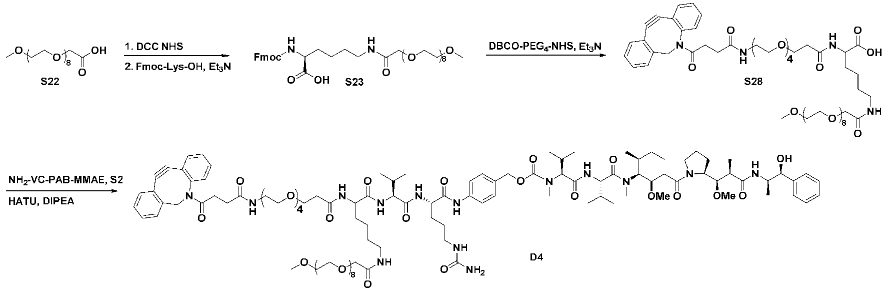

Step 1: **S22** (50 mg, 113.1 µmol) was dissolved in 500 µL of DMF. NHS (39 mg, 339.4 µmol) and DCC (70 mg, 339.4 µmol) were added and the reaction was incubated at 30°C for 2 h. After LC-MS confirmed that the reaction was complete, Fmoc-Lys-OH (41.7 mg, 113.1 µmol) and triethylamine (34.3 mg, 339.4 µmol) were added and the reaction was conducted at 30°C for 3 h. The product was separated and purified using a semi-preparative column and lyophilized to obtain **S23** (64.5 mg, 72% yield). HRMS Calcd. for C₄₀H₆₀N₂O₁₄ [M+H]⁺ 793.4123, found: 793.4137.

Step 2: **S23** (28 mg, 35.4 µmol) was weighed and dissolved in 280 µL of DMF. An equal volume of triethylamine was added and the mixture was reacted at 30°C for 2 h. The reaction mixture was lyophilized and dissolved in 200 µL of DMF. DBCO-PEG₄-NHS (22.8 mg, 35.4 µmol) was added to the above system and the reaction was conducted at 30°C for 3 h. The product was separated and purified using a semi-preparative column and lyophilized to obtain **S28** (22.7 mg, 58% yield). HRMS: Calcd. C₅₅H₈₄N₄O₁₉ [M+H]⁺ 1105.5808, Found: 1105.5289.

Step 3: **S28** (20 mg, 18.1 µmol) was weighed and dissolved in 200 µL of DMF. HATU (13.8 mg, 36.2 µmol), **S2** (20.3 mg, 18.1 µmol), and DIPEA (7.1 mg, 54.3 µmol) were added sequentially. The mixture was reacted at 30°C for 2 h. The product was isolated and purified using a semi-preparative column and lyophilized to obtain **D4** (30.4 mg, 76% yield). HRMS: Calcd. for C₁₁₃H₁₇₆N₁₄O₃₀ [M+2H]²⁺ 1105.6416, [M+3H]³⁺ 737.4303; Found: 1105.6397, 737.4502.

### Example 11: Synthesis of Compound D5

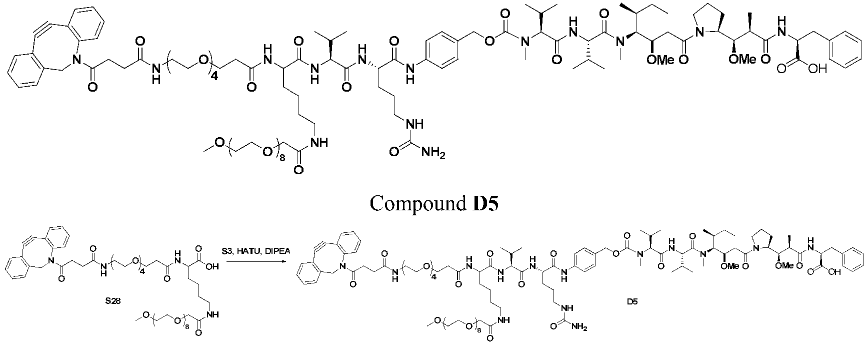

Step 1: S28 (20 mg, 18.1 µmol) was weighed and dissolved in 200 µL of DMF. HATU (8.3 mg, 21.72 µmol), H N-VC-PAB-MMAF (S3, 10.8 mg, 9.5 µmol), and DIPEA (7 mg, 54.3 µmol) were added sequentially. After reacting at room temperature for 1 h, the mixture was separated and purified using a semi-preparative column and lyophilized to obtain **D5** (13.7 mg, 65% yield). HRMS: Calcd. for C₁₁₃H₁₇₄N₁₄O₃₁ [M+2H]²⁺ 1112.6312, [M+3H]³⁺ 742.0901. Found: 1112.6254, 742.1034.

### Example 12: Synthesis of Compound D6

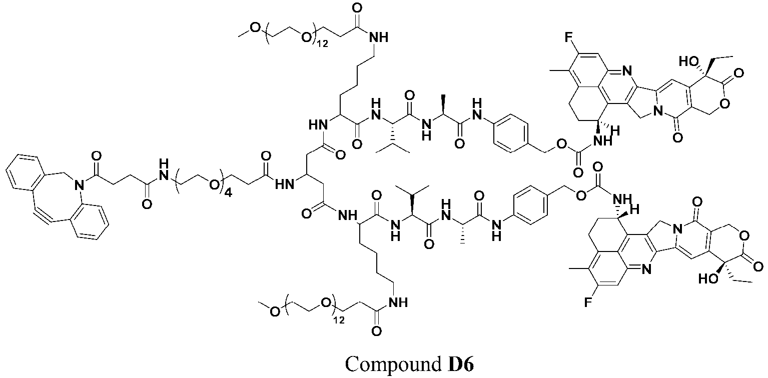

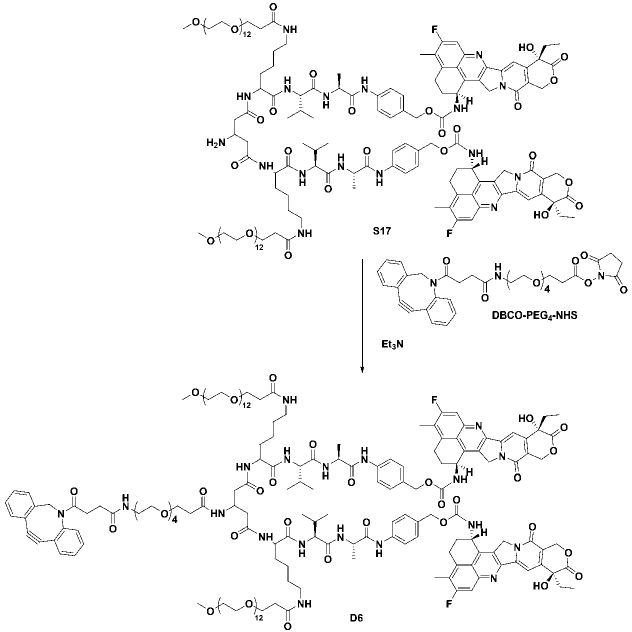

Step 1: DBCO-PEG₄-NHS (20 mg, 18.1 µmol) was weighed and dissolved in 200 µL of DMF. **S17** (56.2 mg, 18.1 µmol) and triethylamine (5.5 mg, 54.3 µmol) were added sequentially. The mixture was reacted at 30°C for 2 h. The product was separated and purified using a semi-preparative column and lyophilized to obtain **D6** (29.8 mg, 66% yield). HRMS: Calcd. for C₁₈₃H₂₅₇F₂N₁₉O₃₃ [M+3H]³⁺ 1213.9366, [M+4H]⁴⁺ 910.7044; Found: 1213.9476, 910.7107.

### Example 13: Synthesis of Compound D7

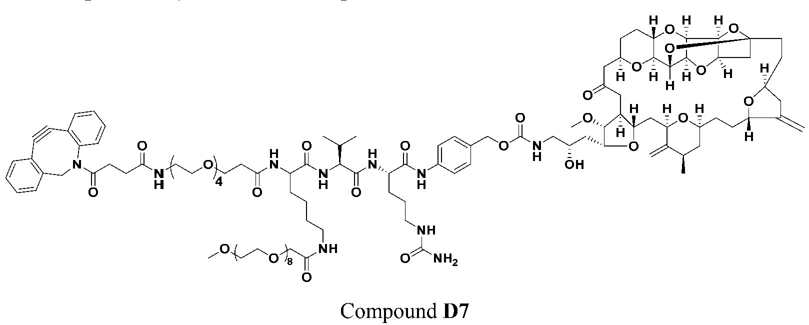

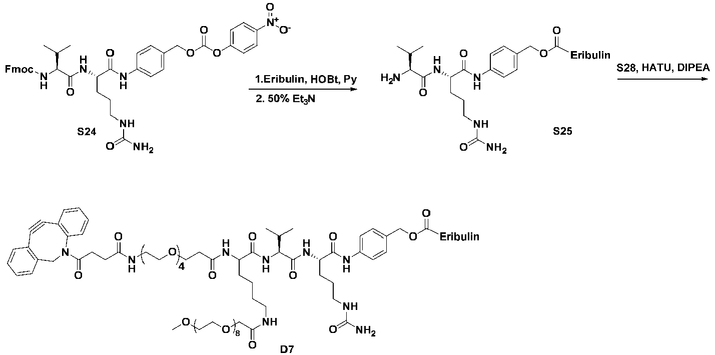

Step 1: Fmoc-VC-PAB-PNP **(S24,** 126.1 mg, 164.4 µmol) was weighed and dissolved in 1.2 mL of DMF. Eribulin (100 mg, 137 µmol), HOBt (27.4 mg, 27.4 µmol), and pyridine (314 µL, 4.1 mmol) were added sequentially. The reaction was allowed to proceed at room temperature overnight. After LC-MS confirmed completion of the reaction, an equal volume of triethylamine was added. The reaction was continued at 30°C for 2 h. The product was separated and purified using a semi-preparative column and lyophilized to obtain S25 (135 mg, 87% yield). HRMS, calcd for C₅₉H₈₆N₆O₁₆ [M+H]⁺ 1135.6178, [M+2H]²⁺ 568.3128, found: 1135.6298, 568.3045.

Step 2: **S28** (58.4 mg, 52.9 µmol) was weighed and dissolved in 200 µL of DMF. HATU (50.3 mg, 132.3 µmol), **S25** (51 mg, 44.1 µmol), and DIPEA (17.1 mg, 132.3 µmol) were added sequentially. The mixture was reacted at 30°C for 2 h. The product was separated and purified using a semi-preparative column and lyophilized to obtain **D7** (57.8 mg, 59% yield). HRMS: Calcd. for C₁₁₄H₁₆₈N₁₉O₃₄ [M+2H]²⁺ 1111.5940, [M+3H]³⁺ 741.3986; Found: 1111.5784, 741.3497.

### Example 14: Synthesis of Compound D8

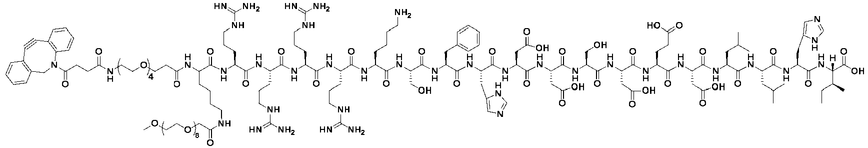

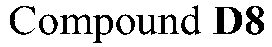

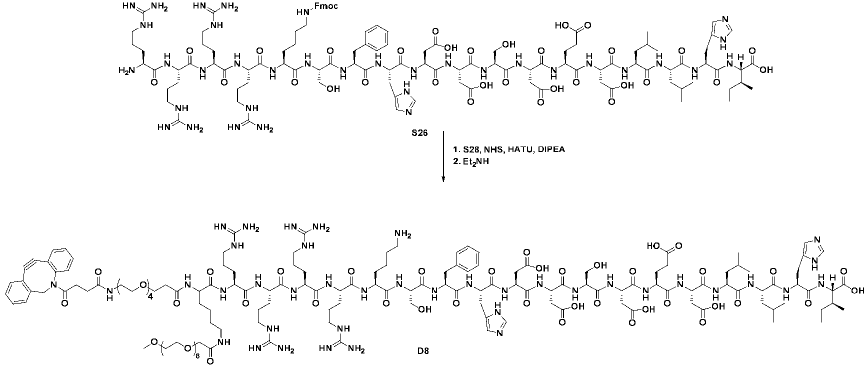

Step 1: **S28** (20 mg, 18.1 µmol) was weighed and dissolved in 200 µL of DMF. NHS (2.5 mg, 21.72 µmol), HATU (8.3 mg, 21.72 µmol), and DIPEA (4.7 mg, 36.2 µmol) were added to the system in sequence. After reacting at room temperature for 1 h, SignalTAC **(S26,** 23.9 mg, 9.5 µmol) and DIPEA (2.3 mg, 18.1 µmol) were added to the system and reacted at 30°C for 2 h. Diethylamine (7 mg, 95 µmol) was added to the reaction system and reacted at 30°C for 3 h. The product was separated and purified using a semi-preparative column to obtain **D8** (13.7 mg, 54% yield). HRMS, Calcd. for C₁₃₁H₂₃₇N₃₉O₄₉ [M+3H]³⁺ 1127.9162, [M+4H]⁴⁺ 846.1891, [M+5H]⁵⁺677.1528, [M+6H]⁶⁺ 564.4620, found: 1127.9238, 846.2126, 677.1782, 564.4285.

### Example 15: Synthesis of Compound D9

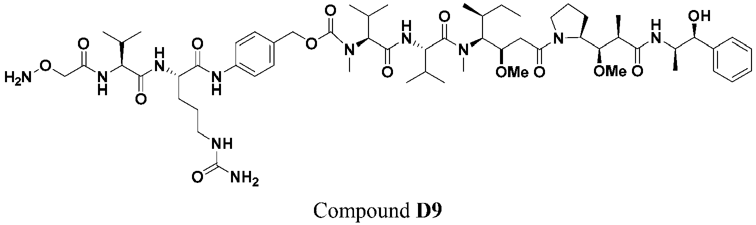

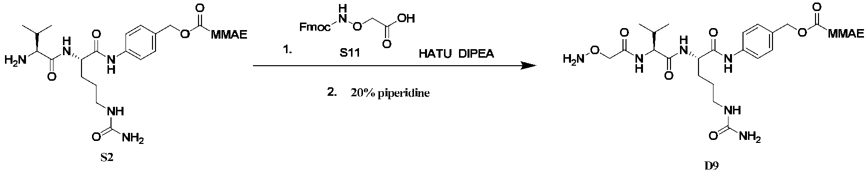

Step 1: **S11** (20 mg, 17.8 µmol) was weighed and dissolved in 200 µL of DMF. HATU (13.5 mg, 35.6 µmol), **S2** (6.7 mg, 21.4 µmol), and DIPEA (93 µL, 53.4 µmol) were added to the reaction system in sequence and mixed. The mixture was reacted at 30°C for 3 h. 124 µL of piperidine was added to the above system, mixed evenly, and reacted at room temperature for 10 min. The product was separated and purified using a semi-preparative column and then lyophilized to obtain **D9** (16 mg, 75 % yield). HRMS, calcd. for C₆₀H₉₇N₁₁O₁₄ [M+H]⁺ 1196.7295, [M+2H]²⁺ 598.8686, found: 1196.7263, 598.8622.

### IV: Synthesis of saccharide chain-directed antibody-functional molecule conjugates GsADC-3a to GsADC-3f

### Example 16: Synthesis of GsADC-3a

The antibody-functional molecule conjugate GsADC-**3a** was obtained from the compound **2a** and the wild-type antibody Herceptin through the general operation I.

### Example 17: Synthesis of GsADC-3b

The antibody-functional molecule conjugate GsADC-**3b** was obtained from the compound **2b** and the wild-type antibody Herceptin through the general operation I.

### Example 18: Synthesis of GsADC-3c

The antibody-functional molecule conjugate GsADC-**3c** was obtained from the compound **2c, D9,** and the wild-type antibody sacituzumab through the general operation VII. The molecular weight found by decomvolution of HRMS was 149025.

### Example 19: Synthesis of GsADC-3d

The antibody-functional molecule conjugate GsADC-**3d** was obtained from the compound **2c, D1,** and the wild-type antibody sacituzumab through the general operation VII. The molecular weight found by decomvolution of HRMS was 159047.

### Example 20: Synthesis of GsADC-3e

The antibody-functional molecule conjugate GsADC**-3e** was obtained from the compound **2c, D9,** and the wild-type antibody hu-Mov-19 through the general operation VII. The molecular weight found by decomvolution of HRMS was 149453.

### Example 21: Synthesis of GsADC-3f

The antibody-functional molecule conjugate GsADC-**3f** was obtained from the compound **2c, D1,** and the wild-type antibody hu-Mov-19 through the general operation VII. The molecular weight found by decomvolution of HRMS was 149481.

V: Synthesis of K248-directed antibody-functional molecule conjugates KsADC-**5a** to KsADC-**5f**

### Example 22: Synthesis of KsADC-5a

The antibody-functional molecule conjugate KsADC-**5a** was obtained from the compound **4a** and the wild-type antibody Herceptin through the general operation II.

### Example 23: Synthesis of KsADC-5b

The antibody-functional molecule conjugate KsADC-**5b** was obtained from the compound 4b and the wild-type antibody Herceptin through general operation II.

### Example 24: Synthesis of KsADC-5c

The antibody-functional molecule conjugate KsADC-**5c** was obtained from the compound **4c, D5**, and the wild-type antibody sacituzumab through the general operation VIII. The molecular weight found by decomvolution of HRMS was 150933.

### Example 25: Synthesis of KsADC-5d

The antibody-functional molecule conjugate KsADC-**5d** was obtained from the compound **4c, D7,** and the wild-type antibody sacituzumab through the general operation VIII. The molecular weight found by decomvolution of HRMS was 150930.

### Example 26: Synthesis of KsADC-5e

The antibody-functional molecule conjugate KsADC-**5e** was obtained from the compound **4c, D5,** and the wild-type antibody hu-Mov-19 through the general operation VIII. The molecular weight found by decomvolution of HRMS was 151372.

### Example 27: Synthesis of KsADC-5f

The antibody-functional molecule conjugate KsADC-5f was obtained from the compound **4c, D7,** and the wild-type antibody hu-Mov-19 through the general operation VIII. The molecular weight found by decomvolution of HRMS was 151368.

### VI: Preparation of bifunctional antibodies Ab-1 to Ab-2

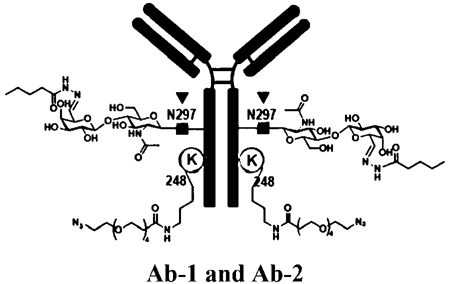

As described in Examples 28 and 29 below, the difference between Ab-1 and Ab-2 lies in the different wild-type antibodies used, thereby obtaining different "bifunctional antibodies".

### Example 28: Synthesis of Ab-1

The bifunctional antibody **Ab-1** was obtained from the compounds **2c** and **4c** and the wild-type antibody sacituzumab through the step 1 of the general operation VI. The molecular weight found by decomvolution of HRMS was 147182.

### Example 29: Synthesis of Ab-2

The bifunctional antibody **Ab-2** was obtained from compounds **2c, 4c** and wild-type antibody hu-Mov-19 through the step 1 of the general operation VI. The molecular weight found by decomvolution of HRMS was 147644.

### VII: Synthesis of Dual-site Antibody-Functional Molecule Conjugates ADC-6a to ADC-6d, ADC-7 to ADC-19

### Example 30: Synthesis of ADC-6a

The antibody-functional molecule conjugate ADC-**6a** was obtained from the compound **2a, 4a** and the wild-type Herceptin through the general operation III. The molecular weight found by decomvolution of HRMS was 151247.

### Example 31: Synthesis of ADC-6b

The antibody-functional molecule conjugate ADC-**6b** was obtained from the compound **2a, 4b** and wild-type Herceptin through the general operation III. The molecular weight found by decomvolution of HRMS was 151273.

### Example 32: Synthesis of ADC-6c

The antibody-functional molecule conjugate ADC-**6c** was obtained from the compound **2b, 4a** and the wild-type Herceptin through the general operation III. The molecular weight found by decomvolution of HRMS was 157215.

### Example 33: Synthesis of ADC-6d

The antibody-functional molecule conjugate ADC-**6d** was obtained from the compound **2b, 4b** and the wild-type Herceptin through the general operation III. The molecular weight found by decomvolution of HRMS was 151302.

### Example 34: Synthesis of ADC-6a-1

The antibody-functional molecule conjugate ADC-**6a-1** was obtained from the compound **2a, 4a** and the wild-type antibody Herceptin through the general operation IV.

### Example 35: Synthesis of ADC-6a-2

The antibody-functional molecule conjugate ADC-**6a-2** was obtained from the compound **2a, 4a** and the wild-type antibody Herceptin the through general operation V.

### Example 36: Synthesis of ADC-7

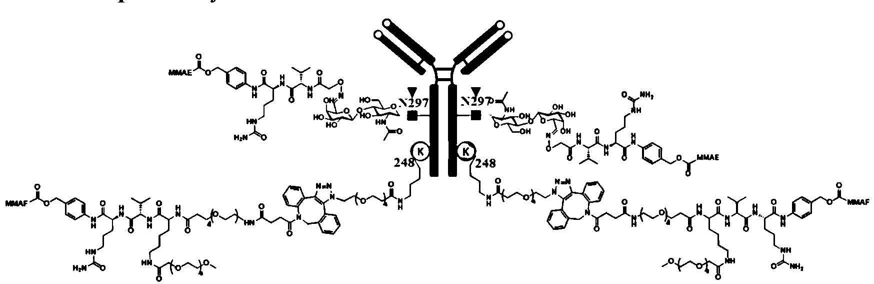

The antibody-functional molecule conjugate ADC-7 was obtained from the compound **D9, D5,** and **Ab-1** through the step 2 of the general operation VI. The molecular weight found by decomvolution of HRMS was 154020.

### Example 37: Synthesis of ADC-8

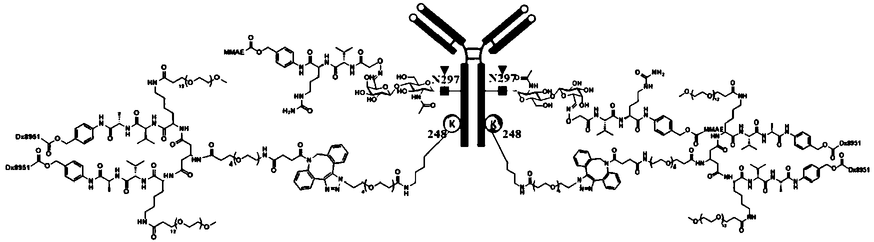

The antibody-functional molecule conjugate ADC-**8** was obtained from the compound **D9, D6,** and **Ab-1** through the step 2 of the general operation VI. The molecular weight found by decomvolution of HRMS was 156858.

### Example 38: Synthesis of ADC-9

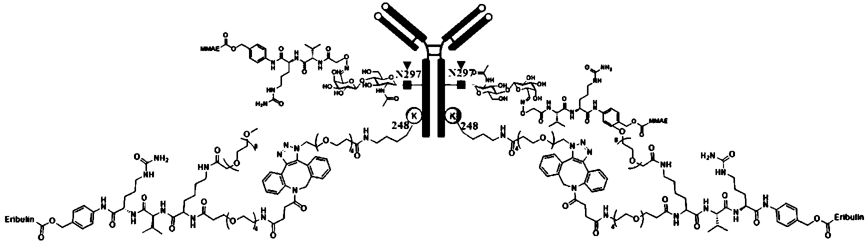

The antibody-functional molecule conjugate ADC-9 was obtained from the compound **D9, D7,** and **Ab-1** through the step 2 of the general operation VI. The molecular weight found by decomvolution of HRMS was 154021.

### Example 39: Synthesis of ADC-10

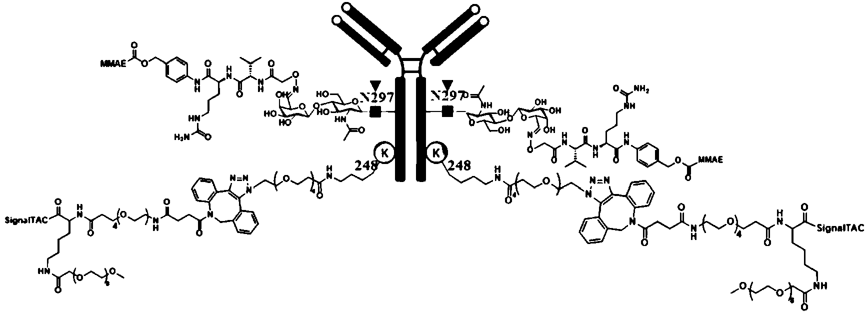

The antibody-functional molecule conjugate ADC-**10** was obtained from the compound **D9, D8,** and **Ab-1** through the step 2 of the general operation VI. The molecular weight found by decomvolution of HRMS was 156341.

### Example 40: Synthesis of ADC-11

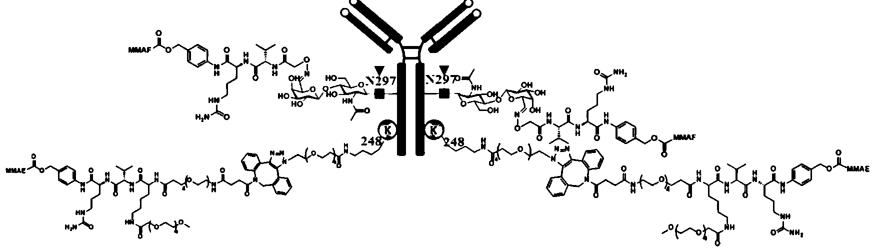

The antibody-functional molecule conjugate ADC-11 was obtained from the compound **D1, D4,** and Ab-1 through the step 2 of the general operation VI. The molecular weight found by decomvolution of HRMS was 154018.

### Example 41: Synthesis of ADC-12

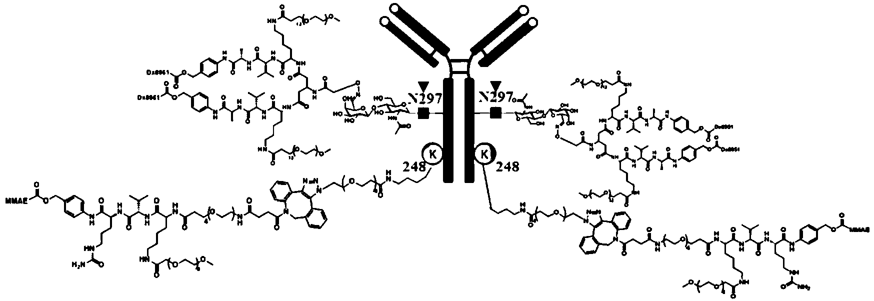

The antibody-functional molecule conjugate ADC-**12** was obtained from the compound **D2, D4,** and **Ab-1** through the step 2 of the general operation VI. The molecular weight found by decomvolution of HRMS was 157958.

### Example 42: Synthesis of ADC-13

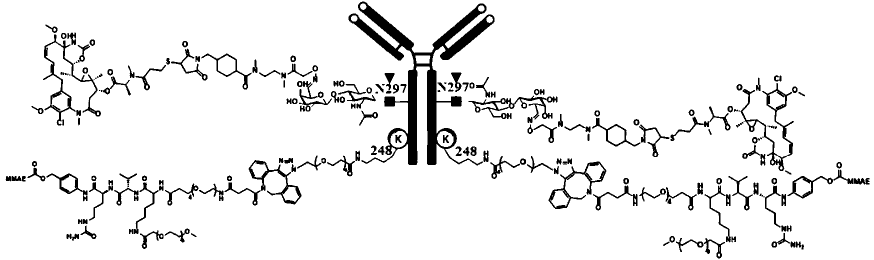

The antibody-functional molecule conjugate ADC-**13** was obtained from the compound **D3, D4,** and **Ab-1** through the step 2 of the general operation VI. The molecular weight found by decomvolution of HRMS was 153835.

### Example 43: Synthesis of ADC-14

The antibody-functional molecule conjugate ADC-**14** was obtained from the compound **D9, D5,** and **Ab-2** through the step 2 of the general operation VI. The molecular weight found by decomvolution of HRMS was 154454.

ADC-**14** differs from ADC-**7** only in the antibody. Since the difference in the antibody is not shown in the structural formula, the structural formula of ADC-**14** is the same as that of ADC-**7** above.

Similarly, the structural formulae of ADC-**15**, ADC-**16**, ADC-**17**, ADC-**18** and ADC-**19** below are the same as those of ADC-**8**, ADC-**9**, ADC-**11**, ADC-**12** and ADC-**13** above, respectively.

### Example 44: Synthesis of ADC-15

The antibody-functional molecule conjugate ADC-**15** was obtained from the compound **D9, D6, and Ab-2** through the step 2 of the general operation VI. The molecular weight found by decomvolution of HRMS was 157282.

### Example 45: Synthesis of ADC-16

The antibody-functional molecule conjugate ADC-**16** was obtained from the compound **D9, D7,** and **Ab-2** through the step 2 of the general operation VI. The molecular weight found by decomvolution of HRMS was 154451.

### Example 46: Synthesis of ADC-17

The antibody-functional molecule conjugate ADC-**17** was obtained from the compound **D1, D4,** and **Ab-2** through the step 2 of the general operation VI. The molecular weight found by decomvolution of HRMS was 154455.

### Example 47: Synthesis of ADC-18

The antibody-functional molecule conjugate ADC-**18** was obtained from the compound **D2, D4,** and Ab**-2** through the step 2 of the general operation VI. The molecular weight found by decomvolution of HRMS was 158386.

### Example 48: Synthesis of ADC-19

The antibody-functional molecule conjugate ADC-**19** was obtained from the compound **D3, D4,** and **Ab-2** through the step 2 of the general operation VI. The molecular weight found by decomvolution of HRMS was 154273.

### Example of in vitro aggregation stability assay

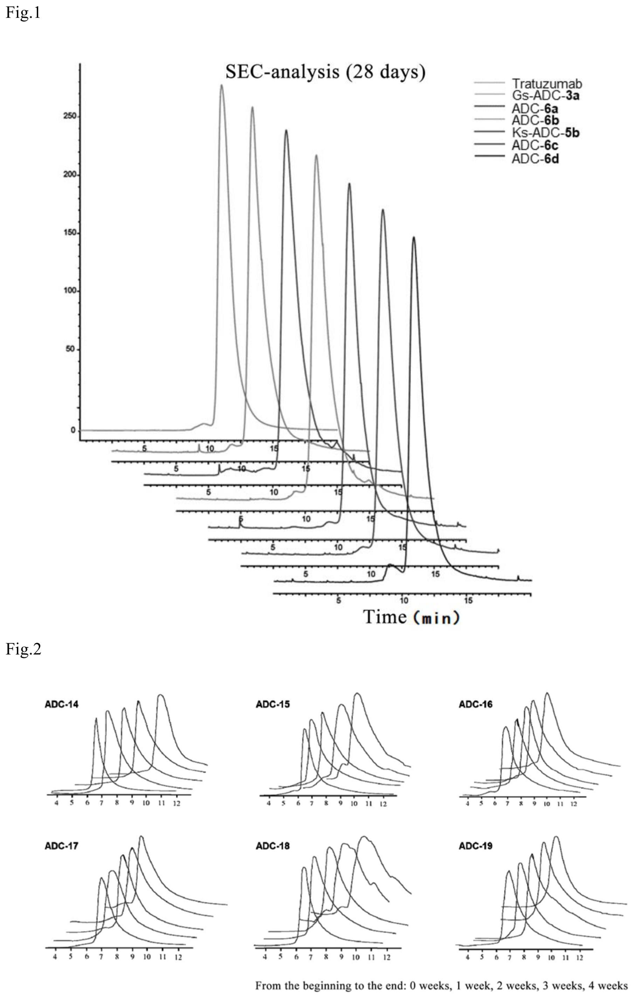

Proteins or antibodies themselves would aggregate due to the change of various chemical or physical factors, forming soluble or insoluble aggregates, and most antibody aggregation is irreversible. Furthermore, the highly hydrophobic nature of the small cytotoxins used in ADC synthesis can lead to hydrophobic interactions between the synthesized ADC molecules, causing the drug aggregation. The specific procedures: 200 mg of each ADC sample was taken and dissolved in 100 mL of PBS and heated at 37°C for 28 days. Aliquots were taken at regular intervals and analyzed using a BioCore SEC-300 column with a particle size of 7.8 × 300 mm, 5 mm, and a gradient of 100% mobile phase (150 mM sodium phosphate, pH 6.8) for 15 min. The absorbance was detected at 280 nm. As shown in Fig. 1, from the size exclusion chromatography (SEC) analysis, the dual-site specific ADCs prepared using technique of the present invention demonstrated excellent stability after 28 days of incubation, without significant protein degradation or aggregation.

### In vitro stability assay based on FRα target

The prepared dual-payload ADC compounds targeting FRα were dissolved in a 1 x PBS buffer system and incubated in a 37°C oven for four weeks. Samples were taken at the time points of 0th, 1st, 2nd, 3rd, and 4th week for SEC column analysis to investigate the aggregation stability of the dual-payload ADC molecules. The results were shown in Fig. 2.

The SEC analytic results showed that the prepared FRα-targeted dual-payload ADCs still showed good aggregation stability after 28 days of incubation, without obvious protein degradation or aggregation.

### Example of uniformity and hydrophilicity assay

### In vitro homogeneity and hydrophilicity assays based on the Trop2 target

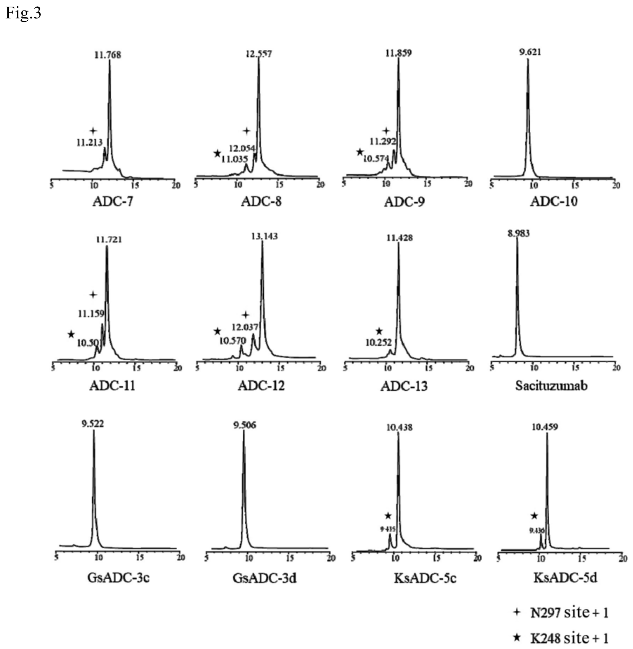

The Trop2-targeting **ADC-7 to ADC-13, GsADC-3c to GsADC-3d, and KsADC-5c to KsADC-Sd,** a total of 11 samples, were diluted with 1 x PBS and analyzed using a reversed-phase chromatography column (PLRP). The injection volume was about 20 µg each time. The results are shown in Fig. 3.

RP data showed that dual-payload ADCs exhibited good homogeneity. Due to the increased number of conjugated payloads, their hydrophobicity was higher than that of single-drug ADCs. Among them, **ADC-7, ADC-9,** and **ADC-13,** which conjugated to MMAF, Eribulin, and DM1 respectively, exhibited similar polarity with retention times of about 11 minutes. **ADC-8** and **ADC-12,** which conjugated to MMAE and Dx8951 respectively, exhibited lower polarity with retention times of about 12-13 minutes, which may be due to the four conjugated Dx8951 toxins. **ADC-10,** which conjugated to MMAE and a lysosome-selective peptide (SignalTAC), exhibited slightly greater polarity with a retention time of about 9 minutes.

### In vitro homogeneity and stability assay based on FRα target

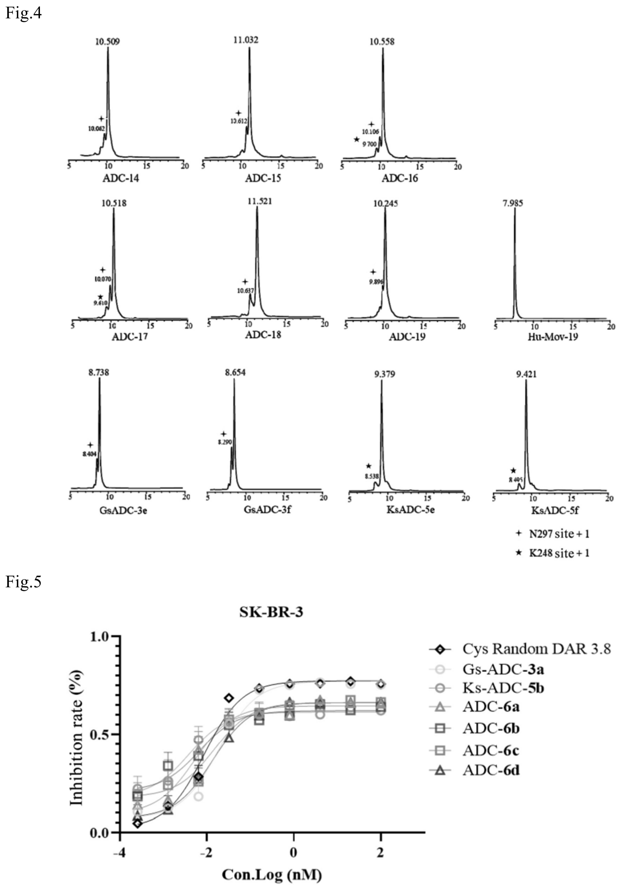

The obtained ADC compounds targeting FRα were diluted with 1 x PBS and analyzed using a reversed-phase chromatography column (PLRP). The injection volume was about 20 µg each time. The results were shown in Fig. 4.

The RP data showed that the prepared dual-payload ADCs all had good uniformity. Compared with single-drug ADCs, the dual-payload ADCs were slightly more hydrophobic, with a retention time of 10-11 minutes.

### Pharmacological Example 1

### Experimental process and results analysis of in vitro activity data based on HER2 target

The dual-site specific ADCs described above were evaluated for cellular activity. Three cell lines were selected, including SK-Br-3 cells and NCI-N87 cells, which are Her2-positive cells, and MDA-MB-231 cells, which are Her2-negative cells. The MTT assay was used to test the cellular activity and cytotoxicity of the ADC molecules.

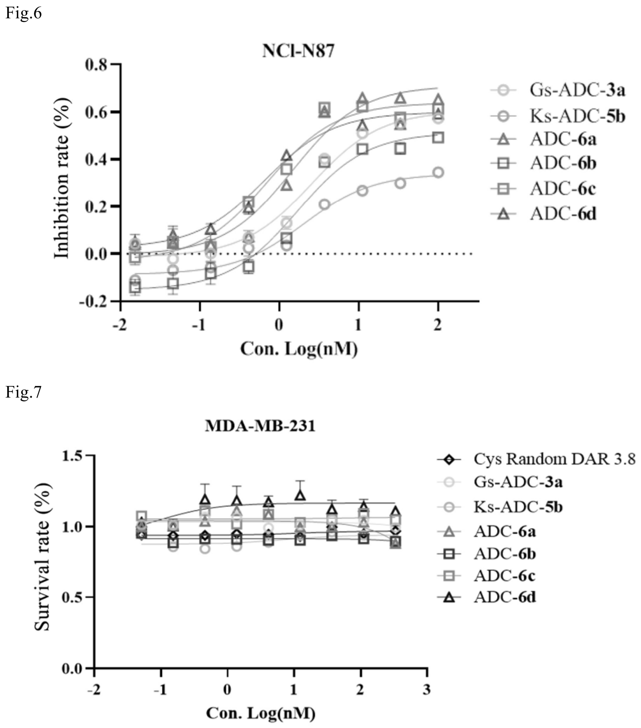

The specific procedure was as follows: 100 mL of PBS was added to the outermost wells of a 96-well plate. Three other wells were filled with culture medium only. About 6,000 cells of the corresponding type were added to each of the remaining wells and incubated overnight at 37°C in a CO₂ incubator. 10 mL of each ADC molecule was added (each ADC molecule was serially diluted fivefold, starting from a maximum concentration of 100 nM, for a total of nine concentrations, with three replicates per well). 10 µL of culture medium was added to the remaining three wells containing cells and the three wells containing culture medium only, serving as control and blank groups. The 96-well plate was incubated at 37°C in a CO₂ incubator for 72 hours. 10 mL of 5 mg/mL MTT was added to each well and incubated at 37°C for 4 hours. Subsequently, 90 mL of SDS lysis buffer was added to each well and incubated at 37°C for 7 hours to fully lyse the cells. Finally, the OD value of each well was measured at 570 nm, and data were processed using GraphPad Prism 8. The results are shown in Figs. 5-7. The dual-site directed antibody-functional molecule conjugates showed strong in vitro cell activities without toxicity in negative cells.

### Pharmacological Example 2

### Experimental process and results analysis of in vitro activity data based on Trop2 target

The Trop2-targeted dual-site specific ADCs mentioned above were evaluated for cellular activity. Four cell lines were selected, including BxPC-3 cells, which are high-Trop2-expressing cells, Colo-205 and Capan-1 cells, which are medium-Trop2-expressing cells, and MDA-MB-231 cells, which are low-Trop2-expressing cells. The MTT assay was used to test the cellular activity and toxicity of the ADC molecules.

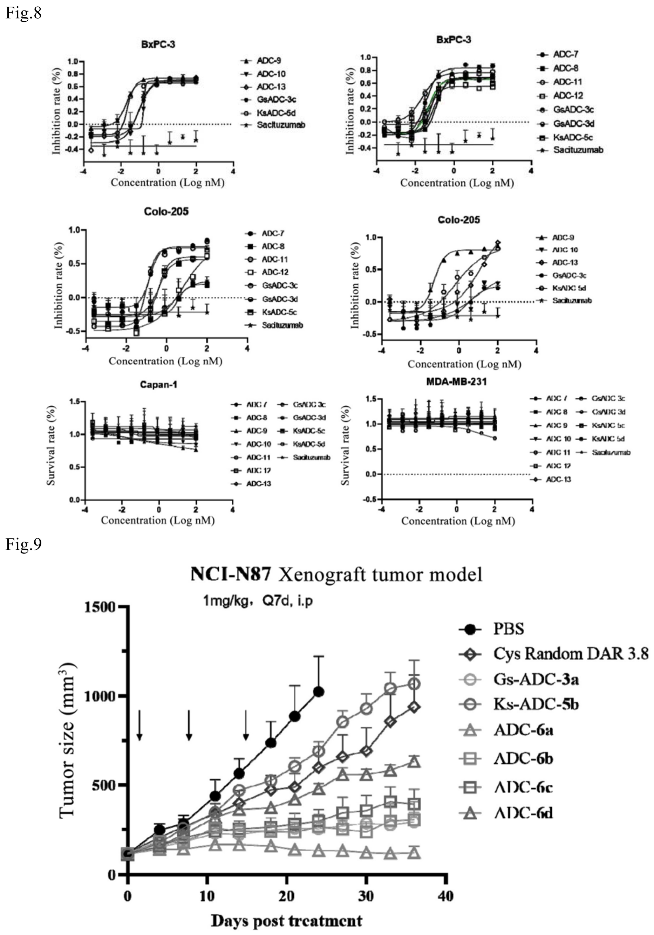

The specific procedure was as follows: 100 mL of PBS was added to the outermost wells of a 96-well plate. Three other wells were filled with culture medium only. About 6,000 cells of the corresponding type were added to each of the remaining wells and incubated overnight at 37°C in a CO₂ incubator. 20 µL of each ADC molecule was added (each ADC molecule was serially diluted fivefold, starting from a maximum concentration of 100 nM, for a total of nine concentrations, with three replicates per well). 20 µL of culture medium was added to the remaining three wells containing cells and the three wells containing culture medium only, serving as control and blank groups, for a total of 200 µL per well. The 96-well plate was then incubated in a CO₂ incubator at 37°C for 144 hours. The culture medium was replaced with 100 µL of fresh culture medium, and 10 µL of 5 mg/mL MTT was added to each well. The cells were then incubated at 37°C for 4 hours. Subsequently, 90 µL of SDS lysis buffer was added to each well and incubated at 37°C for 7 hours to fully lyse the cells. Finally, the OD value of each well was measured at 570 nm, and data were processed using GraphPad Prism 8. The results are shown in Fig. 8.

The dual-site directed antibody-functional molecule conjugates showed strong in vitro cellular activities without toxicity in negative cells. In the high-expressing cell line BxPC-3, the dual-site specific directed ADC compounds showed comparable in vitro inhibitory activity to single-drug ADCs. In the medium-expressing cell line Colo-205, the combinations of MMAE(N)-MMAF(K), MMAE(N)-Eribulin(K), and MMAF(N)-MMAE(K) **(ADC-7, ADC-9,** and **ADC-11)** showed enhanced cellular inhibitory activities. However, the combinations of MMAE and Dx8951 **(ADC-8** and **ADC-12),** the combination of MMAE and SignalTAC **(ADC-10),** and the combination of DM1 and MMAE **(ADC-13)** did not exert good inhibitory effects, possibly because the Dx8951 toxin is insensitive to these cells, the amount of lysosomal targeting peptide SignalTAC that exerts a targeting effect is too low, and DM1 uses a non-cleavable linkage.

### Pharmacological Example 3:

### In vivo anti-tumor activity experimental process and results analysis based on HER2 target

The gastric cancer cell line NCI-N87 was used to establish a BALB/c nude mouse xenograft tumor model and mice were grouped using ear hole marking and large, medium and small grouping principle, with 6 mice in each group.

A disaccharide ADC compound Gs-ADC-**3a**, an ADC compound Ks-ADC-**5b** which is synthesized by the affinity fragment-directed technique, and four dual-site specific antibody ADC compounds, ADC-**6a** to ADC-**6d**, were evaluated for activity in animals. A Cys-randomly conjugated ADC compound (DAR ≈ 3.8) served as a positive control, and PBS served as a negative control. All samples were diluted to 0.1 mg/mL in 1x PBS and sterilized using a 0.22 mm filter prior to administration.

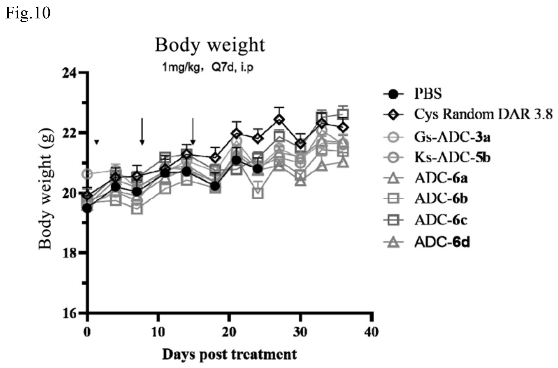

All samples were administered intraperitoneally at 1 mg/kg, once every seven days for a total of three doses. Tumor size and mouse body weight were recorded using a vernier caliper every three days starting from the first dose. The experimental procedures adhered to animal ethics standards. The measured data were graphically analyzed using GraphPad Prism 8 software. As shown in Figs. 9-10, the dual-site directed ADCs prepared using the techniques of the present invention exhibited excellent in vivo activities without significant toxicity.

## Claims

1. A dual-site directed antibody-functional molecule conjugate comprising
an antibody,
linker fragments 1 and 2, and
a functional molecule;
wherein the functional molecule is conjugated to a conserved glycosylation site in the Fc region of the antibody via the linker fragment 1, and to the lysine 246 or 248 of the antibody via the linker fragment 2.

2. The dual-site directed antibody-functional molecule conjugate according to claim 1, wherein
the functional molecule is conjugated to the conserved glycosylation site in the Fc region of the antibody via the linker fragment 1, and to the lysine site 248 of the antibody via the linker fragment 2.

3. The dual-site directed antibody-functional molecule conjugate according to claim 1, wherein
the antibody is selected from the group consisting of humanized antibodies comprising an Fc region or other animal-origin antibodies comprising an Fc region;
the functional molecules are the same as or different from each other and are independently selected from the group consisting of reactive functional groups, fluorescent groups, drugs, toxins, radioactive structures, and lysosomal targeting peptides;
the linker fragment 1 comprises a linker and a saccharide structure, wherein the saccharide structure is linked to the conserved glycosylation site in the Fc region of the antibody, and the linker is linked to the functional molecule;
the linker fragment 2 comprises a linker and a fragment A, wherein the fragment A is linked to the lysine 246 or 248 of the antibody, and the linker is linked to the functional molecule;
wherein the saccharide structure represents a structure of a monosaccharide, a disaccharide, an oligosaccharide or a branched saccharide; the fragment A represents an amide structure or a triazole structure; at the glycosylation site of the antibody Fc domain, the linker represents any structure that can link the saccharide structure and the functional molecule; and at the lysine 246 or 248 of the antibody, the linker represents any structure that can link the fragment A and the functional molecule.

4. The dual-site directed antibody-functional molecule conjugate according to claim 3, wherein
the antibody is selected from the group consisting of antibodies of different IgG subtypes, monoclonal antibodies, bifunctional or multifunctional antibodies, polyclonal antibodies, and functional antibodies of different species;
in the functional molecule, the reactive functional group is selected from the group consisting of an azide group, a tetrazine group, a TCO group, an alkynyl structure, an aldehyde structure, a carbonyl structure, a hydroxylamine structure, an isothiocyanate, an amino group, a carboxyl group, a mercapto group, an alkenyl group, a maleimide, an acylhydrazone structure;
the fluorescent group is selected from the group consisting of biotin, FITC, rhodamine, Cy3, and Cy5;
the drug or toxin is selected from the group consisting of MMAE, MMAF, Dxd, Dx8951, SN38, DM1, DM4, PBD, PBD dimer, eribulin, duocarmycin and derivatives of these drugs;
the radioactive structure is selected from the group consisting of ⁶⁸Ga, ¹⁸F, ⁸⁹Zr;
the lysosomal targeting peptide is SignalTAC.

5. The dual-site directed antibody-functional molecule conjugate according to claim 1, wherein the dual-site directed antibody-functional molecule conjugate is represented by the following formula I: 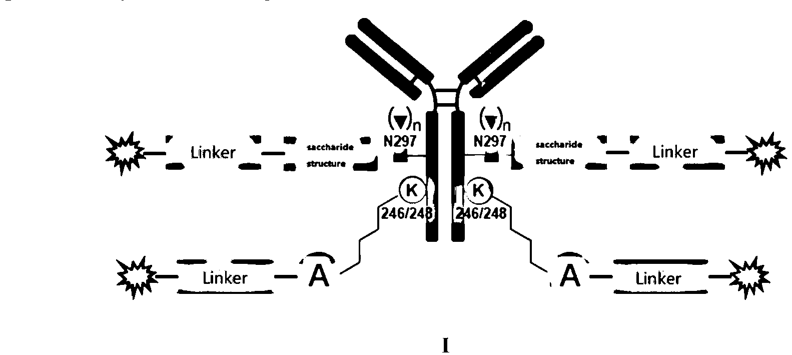
in Formula I,
the "Y"-shaped structure in the center is an antibody;
▼ represents a core fucose structure;
n is 0 or 1;
the linker represents a PEG chain, a carbon chain, a cleavable linker;
the saccharide structure represents a monosaccharide, a disaccharide, an oligosaccharide or a branched saccharide structure;
the fragment A represents an amide structure or a triazole structure;
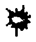 represents a functional molecule, and the functional molecules at the glycosylation site and the lysine site can be the same or different, and one or more functional molecules can be conjugated.

6. The dual-site directed antibody-functional molecule conjugate according to claim 5, wherein the dual-site directed antibody-functional molecule conjugate of Formula I is represented by the following Formula II: 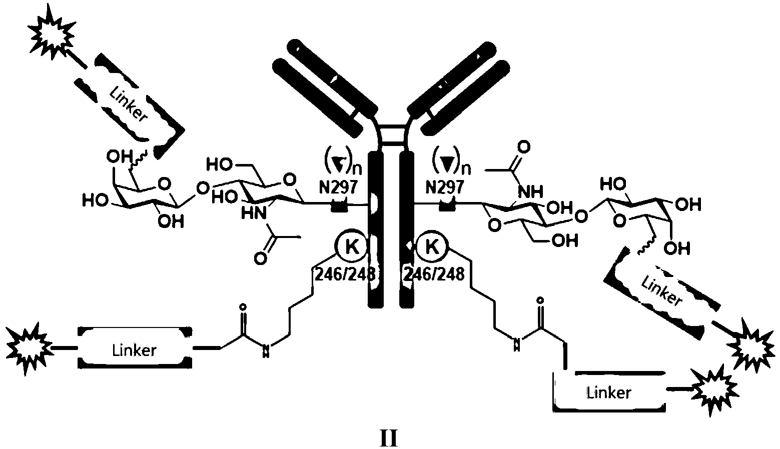 in Formula II, the definitions of the respective moieties are the same as those in claim 5.

7. The dual-site directed antibody-functional molecule conjugate according to claim 5, wherein the dual-site-directed antibody-functional molecule conjugate of Formula II is selected from the group consisting of: 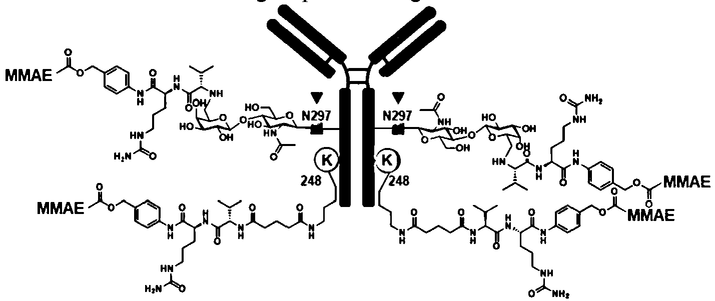 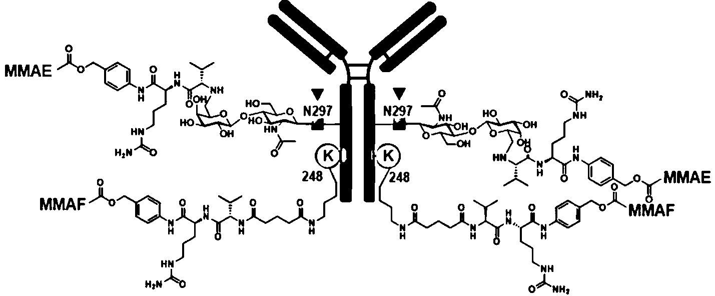 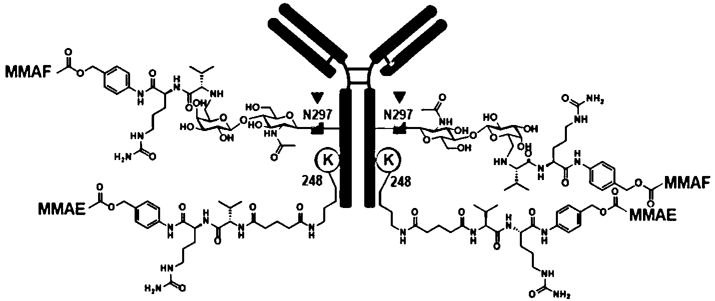 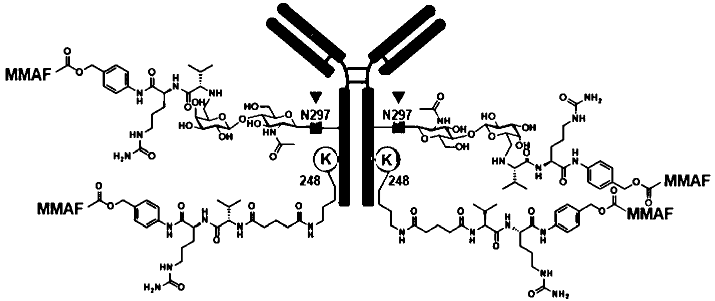 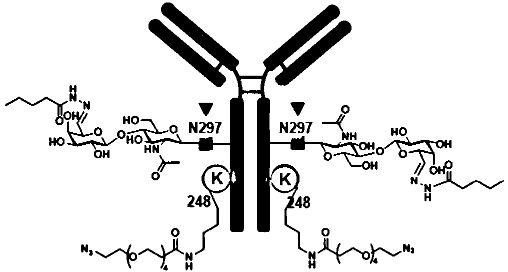 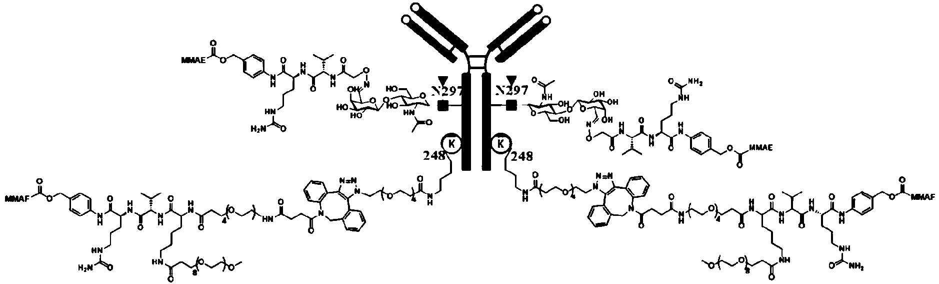 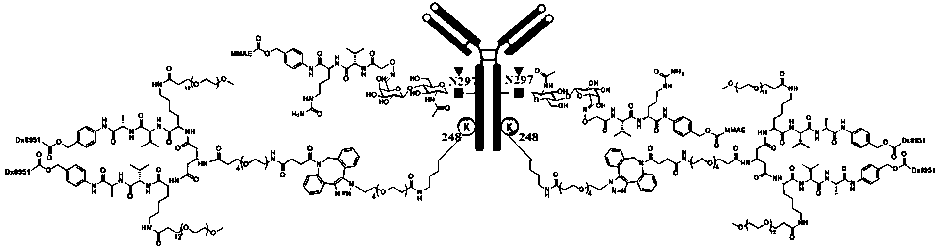 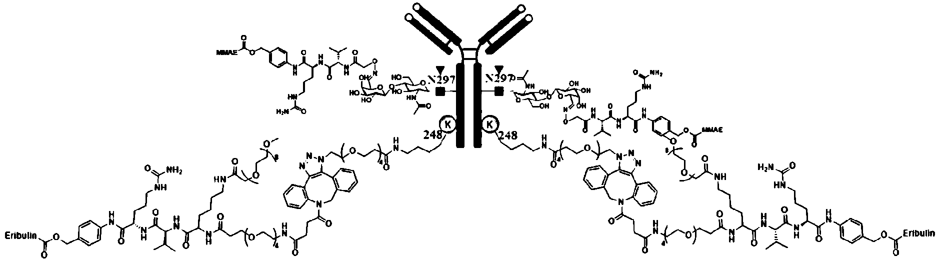 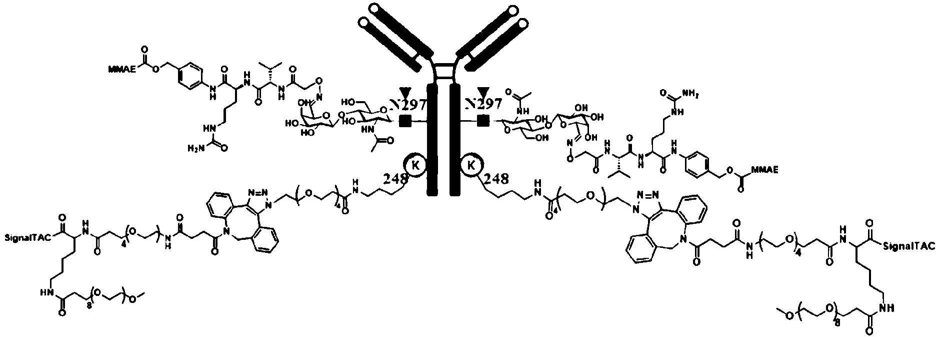 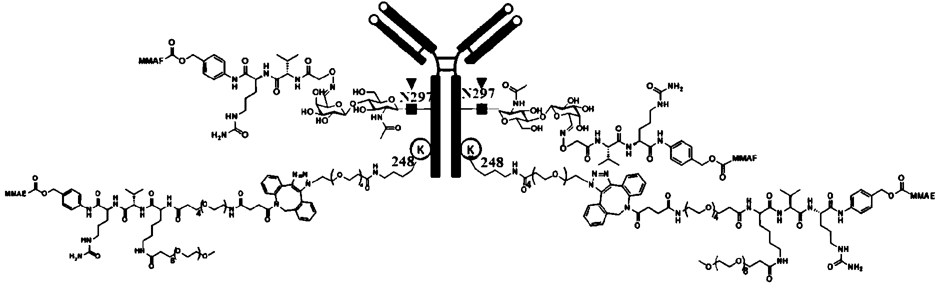 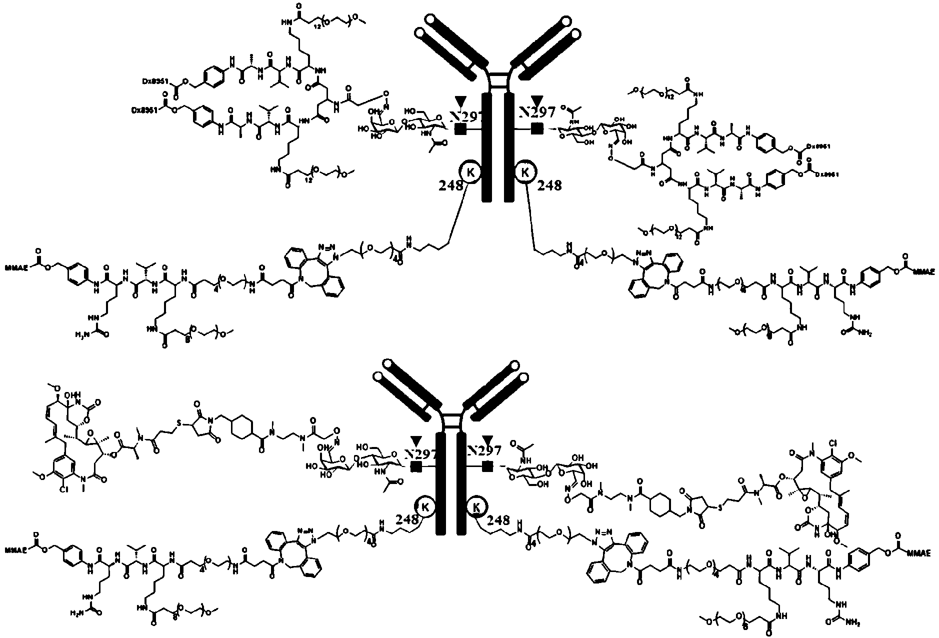 wherein,
MMAE has a structure of 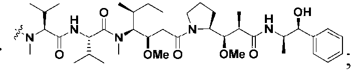
MMAF has a structure of 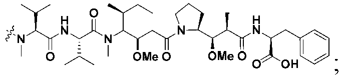
Dx8951 has a structure of 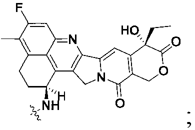
Eribulin has a structure of 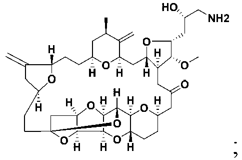
SignalTAC has a structure of

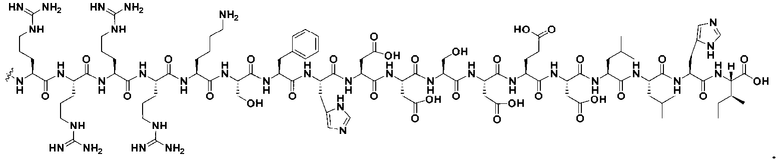

8. A method for preparing a dual-site antibody-functional molecule conjugate, which is selected from the group consisting of the following methods I to III, as shown in the following reaction formula: 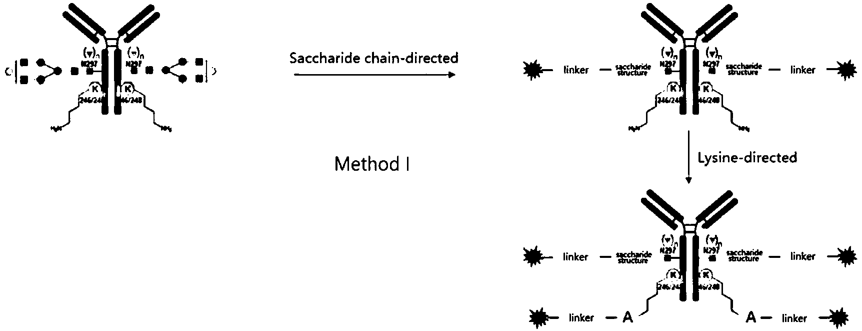 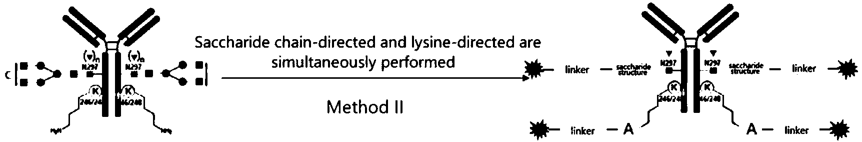 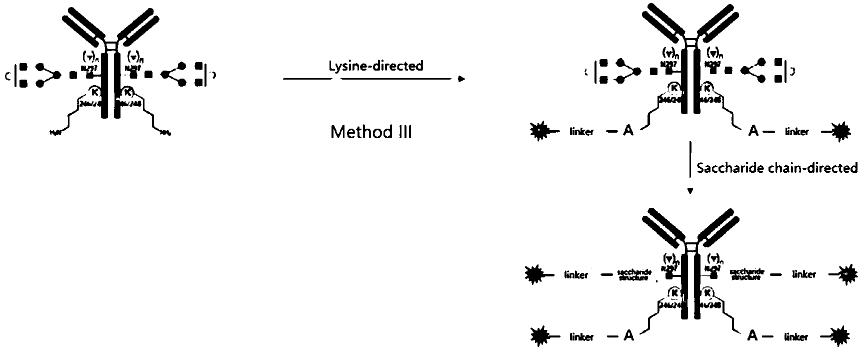
wherein, in the above reaction formula, the definitions of various moieties are the same as those in claim 5,
Method I:
subjecting a wild-type antibody or a defucosylating antibody to a saccharide chain-directed technique mediated by a glycosyltransferase or endoglycosidase to obtain a saccharide chain-directed antibody-functional molecule conjugate with a functional molecule modified at the glycosylation site of the antibody,
subsequently conjugating a functional molecule-linker to the lysine 246 or 248 of the antibody through a targeting peptide/protein-mediated K246 or K248 site-directed technique to finally obtain a dual-site modified antibody-functional molecule conjugate;
Method II:
to the wild-type antibody or defucosylating antibody, adding an enzyme and a substrate required for saccharide chain-directed technique and a substrate required for targeting peptide/protein-mediated lysine-directed technique at the same time, and incubating to obtain a dual-site modified antibody-functional molecule conjugate;
Method III:
first subjecting a wild-type antibody or a defucosylating antibody to a targeting peptide/protein-mediated K246 or K248 site-directed technique to conjugate a functional molecule-linker at the lysine 246 or 248 of the antibody,
subsequently using a glycosyltransferase or endoglycosidase-mediated saccharide chain-directed technique to conjugate a functional molecule at the glycosylation site of the antibody to finally obtain a dual-site modified antibody-functional molecule conjugate;
wherein, the glycosyltransferase is selected from the group consisting of fucosyltransferase, galactosyltransferase, and sialyltransferase, and the endoglycosidase is selected from the group consisting of Endo-S, Endo-S2, Endo-F3, Endo-M and mutants of these enzymes, and includes fusion proteins formed by fusing the functional regions of these different endoglycosidases.

9. The method according to claim 8, wherein
the Method I includes:
Step a1: placing the antibody in a buffer solution, adding a saccharide structure-functional molecule complex and a glycosyltransferase or endoglycosidase, and reacting at a temperature of 15°C-37°C for 30 minutes to 20 hours to obtain a saccharide chain-directed antibody-functional molecule conjugate with functional molecule modified at glycosylation site of the antibody,
Step a2: then placing the product obtained in step a1 in a buffer solution, adding a targeting peptide-functional molecule complex, reacting the mixture at a temperature of 0°C-40°C for 30 minutes to 20 hours to conjugate the functional molecule-linker at the lysine 246 or 248 of the antibody, finally obtaining a dual-site modified antibody-functional molecule conjugate;
the method II includes:
placing an antibody in a buffer solution, adding a glycosyltransferase or endoglycosidase and a saccharide structure-functional molecule complex, and simultaneously adding a targeting peptide-functional molecule complex, reacting at a temperature of 15°C-37°C for 30 minutes to 20 hours, and obtaining a dual-site modified antibody-functional molecule conjugate;
the method III includes:
Step c1: placing an antibody in a buffer solution, adding a targeting peptide-functional molecule complex, and reacting at a temperature of 0°C-40°C for 30 minutes to 20 hours to conjugate the functional molecule-linker at the lysine 246 or 248 of the antibody,
Step c2: then placing the product obtained in step c1 in a buffer solution, adding a glycosyltransferase or endoglycosidase and a saccharide structure-functional molecule complex, reacting at a temperature of 15°C-37°C for 30 minutes to 20 hours to finally obtain a dual-site modified antibody-functional molecule conjugate.

10. The method according to claim 8, wherein the dual-site antibody functional molecule conjugate is prepared by method I or method III,
wherein in step a1 of method I and step c2 of method III, a endoglycosidase-mediated saccharide chain site-directed technique is used; in step a2 of method I and step c1 of method III, an Fc-binding peptide is used as the Fc domain-directed fragment of the antibody, and a thioester structure is used as an acyl transfer fragment.

11. A method for preparing a dual-site antibody-functional molecule conjugate, which is selected from the group consisting of method IV and method V, as shown in the following reaction formula:
Method IV: 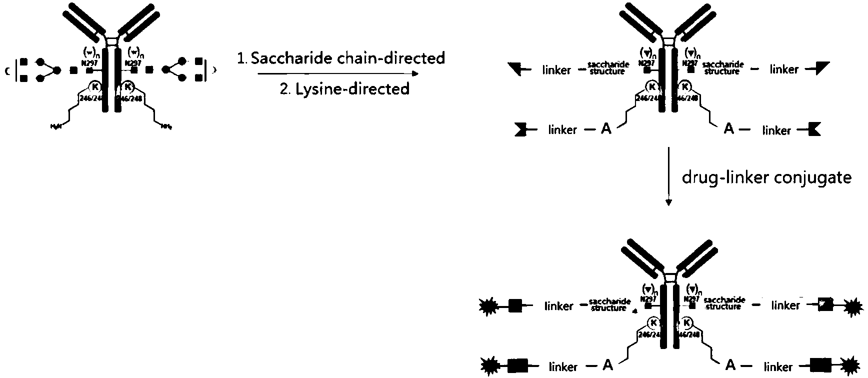 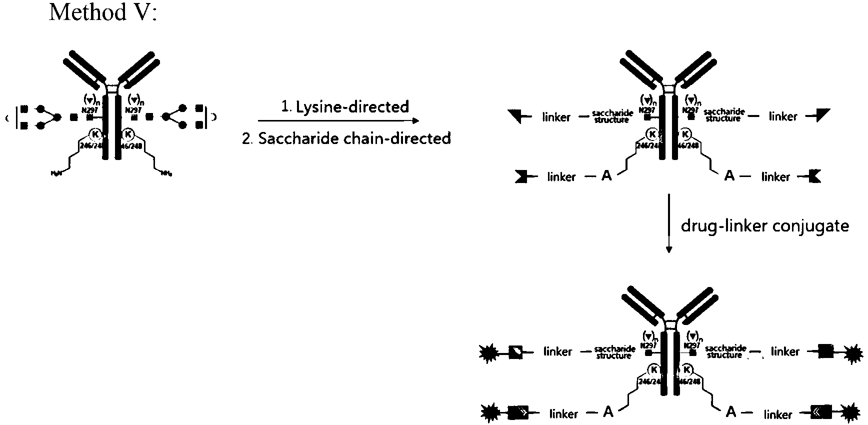 in the above reaction formula, the definitions of various moieties are the same as those in claim 5, and " 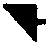 " and " 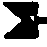 " represent reactive functional groups:
Method IV:
subjecting a wild-type antibody or a defucosylating antibody to a glycosyltransferase or endoglycosidase-mediated saccharide chain-directed technique to obtain a saccharide chain-directed antibody-reactive functional group conjugate with a functional molecule modified at the glycosylation site of the antibody,
subsequently conjugating a reactive functional group-linker to the lysine 246 or 248 of the antibody through a targeting peptide- or targeting protein-mediated K246 or K248 site-directed technique to obtain a dual-site modified bifunctional antibody,
subsequently introducing functional molecules into the glycosylation site and the K246 or K248 site through a bioorthogonal reaction, finally obtaining a dual-site modified antibody-functional molecule conjugate;
Method V:
first conjugating a wild-type antibody or a defucosylating antibody to a reactive functional group-linker at the lysine 246 or 248 of the antibody through a targeting peptide- or targeting protein-mediated K246 or K248 site-directed technique,
subsequently conjugating a reactive functional group to the glycosylation site of the antibody through a glycosyltransferase or endoglycosidase-mediated saccharide chain-directed technique to obtain a dual-site modified bifunctional antibody,
subsequently introducing functional molecules at the glycosylation site and the K246 or K248 site respectively through a bioorthogonal reaction to finally obtain a dual-site modified antibody-functional molecule conjugate,
wherein the glycosyltransferase is selected from the group consisting of fucosyltransferase, galactosyltransferase, and sialyltransferase; the endoglycosidase is selected from the group consisting of Endo-S, Endo-S2, Endo-F3, Endo-M, and mutants of these enzymes, and comprises a fusion protein formed by fusing the functional regions of these different endoglycosidases; and the reactive functional group is defined as that in claim 4.

12. The method according to claim 11, wherein
the method IV comprises:
Step b1: placing an antibody in a buffer solution, adding a saccharide structure-reactive functional group complex and a glycosyltransferase or endoglycosidase, and reacting at a temperature of 15°C-37°C for 30 minutes to 20 hours to obtain a saccharide chain-directed antibody-reactive functional group conjugate with a reactive functional group modified at the glycosylation site of antibody; then adding a targeting peptide-reactive functional group complex to the above system and reacting at a temperature of 0°C-40°C for 30 minutes to 20 hours to conjugate the reactive functional group-linker at the lysine 246 or 248 of the antibody to obtain a dual-site modified bifunctional antibody,
Step b2: subsequently adding two functional molecules-linkers to the buffer system of step b1, and reacting at a temperature of 0°C-40°C for 30 minutes to 20 hours to introduce different or same functional molecules at the glycosylation site and the lysine 246 or 248 of the antibody, finally obtaining a dual-site modified antibody-functional molecule conjugate;
the method V comprises:
Step d1: placing an antibody in a buffer solution, adding a targeting peptide-reactive functional group complex, and reacting at a temperature of 0°C-40°C for 30 minutes to 20 hours to obtain a lysine-site-directed antibody-reactive functional group conjugate with a reactive functional group modified at the lysine 246 or 248 of the antibody; then adding a saccharide structure-reactive functional group complex and a glycosyltransferase or endoglycosidase to the above system, and reacting at a temperature of 15°C-37°C for 30 minutes to 20 hours to conjugate the reactive functional group-linker at the glycosylation site of the antibody to obtain a dual-site modified bifunctional antibody,
Step d2: then adding two functional molecule-linkers to the buffer system of step d1 and reacting at a temperature of 0°C-40°C for 30 minutes to 20 hours to introduce different or same functional molecules at the glycosylation site and the lysine 246 or 248 of the antibody, finally obtaining a dual-site modified antibody-functional molecule conjugate.

13. The method according to claim 11, wherein the method is carried out by the following method VI,
Method VI: 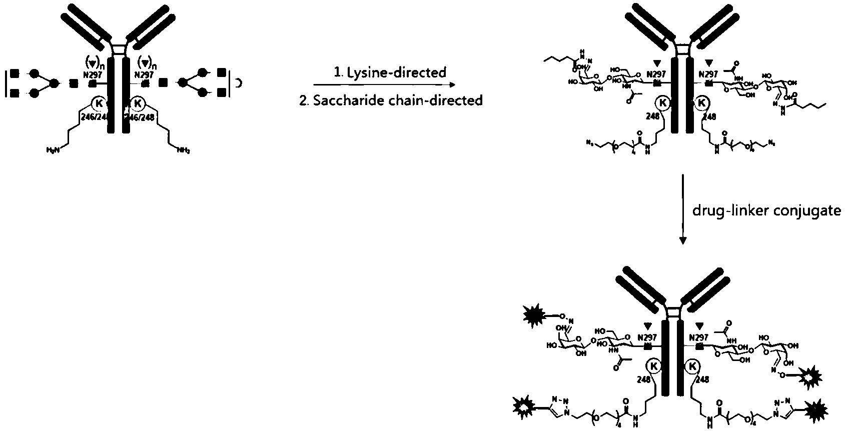 in the above reaction formula, the definitions of various moieties are the same as those in claim 5,
Method VI:
Subjecting a wild-type antibody or a defucosylated antibody to a saccharide chain-directed technique mediated by endoglycosidase to obtain a saccharide chain-directed antibody-reactive functional group conjugate with an acylhydrazone structure modified at the glycosylation site of the antibody,
subsequently conjugating an azide functional group to the lysine 246 or 248 of the antibody through a targeting peptide- or targeting protein-mediated K246 or K248 site-directed technique to obtain a bifunctional antibody with dual-site modification of acylhydrazone and azide; then introducing functional molecules at the glycosylation site and the K246 or K248 site respectively through acylhydrazone-oxime exchange chemistry and click chemistry reactions to finally obtain a dual-site modified antibody-functional molecule conjugate.

14. The method according to claim 13, wherein the method comprises:
Step e1: placing an antibody in a pH 6.0-8.0 buffer, adding a saccharide structure-reactive functional group complex and a glycosyltransferase or endoglycosidase, and reacting at a temperature of 15°C-37°C for 30 minutes to 20 hours to obtain a saccharide chain-directed antibody-reactive functional group conjugate with a functional molecule modified at the glycosylation site of the antibody; then adding a targeting peptide-reactive functional group complex to the above system, using different concentrations of organic solvents to assist solubilization, and reacting at a temperature of 0°C-40°C for 30 minutes to 20 hours to conjugate the reactive functional group-linker at the lysine 246 or 248 of the antibody to obtain a dual-site modified bifunctional antibody,
Step e2: then adding two functional molecule-linkers and a catalyst for catalyzing acylhydrazone-oxime exchange chemistry to the buffer system of step e1, and reacting at a temperature of 0°C-40°C for 30 minutes to 20 hours to introduce different or same functional molecules at the glycosylation site and the lysine 246 or 248 of the antibody, finally obtaining a dual-site modified antibody-functional molecule conjugate.

15. Use of the dual-site directed antibody-functional molecule conjugate according to any one of claims 1 to 7 in preparation of a drug, a diagnostic and therapeutic reagent, or a kit, wherein the drug, diagnostic and therapeutic reagent, and kit are used to treat a tumor, a disease caused by inflammation, a disease caused by viral infection, or an immune disease.

16. The use according to claim 15, wherein
the tumor is selected from the group consisting of ovarian cancer, breast cancer, fallopian tube cancer, endometrial cancer, peritoneal cancer, gastric cancer, colon cancer, bladder cancer, pancreatic cancer, biliary tract cancer, osteosarcoma, cervical cancer, head and neck tumors, germ cell and embryonal cancer, esophageal cancer, malignant glioma, Ewing sarcoma, melanoma, bile duct cancer, prostate cancer, small cell lung cancer, non-small cell lung cancer, lymphoma and blood cancer;
the disease caused by inflammation is selected from the group consisting of conjunctivitis, bronchitis, sinusitis, and Crohn's disease;
the disease caused by the viral infection is selected from the group consisting of influenza, herpes zoster, human papillomavirus infection, AIDS, hepatitis A, hepatitis B, chickenpox;
the immune disease is selected from the group consisting of rheumatoid arthritis, systemic lupus erythematosus, myasthenia gravis, myeloma, dermatomyositis, and psoriasis.
